(19) Europäisches Patentamt
European Patent Office
Office européen des brevets

(11) EP 4 311 555 A1

(12) EUROPEAN PATENT APPLICATION
published in accordance with Art. 153(4) EPC

(43) Date of publication:
**31.01.2024 Bulletin 2024/05**

(21) Application number: **22775729.1**

(22) Date of filing: **23.03.2022**

(51) International Patent Classification (IPC):
***A61K 38/43*** (2006.01) ***A61K 47/34*** (2017.01)
***A61J 1/05*** (2006.01) ***A61K 47/65*** (2017.01)
***A61K 9/08*** (2006.01) ***A61K 47/68*** (2017.01)
***A61K 9/19*** (2006.01) ***A61P 3/00*** (2006.01)
***A61K 38/47*** (2006.01) ***A61P 43/00*** (2006.01)
***A61K 39/395*** (2006.01) ***C07K 16/28*** (2006.01)
***A61K 45/00*** (2006.01) ***C07K 16/46*** (2006.01)
***A61K 47/02*** (2006.01) ***C12N 9/24*** (2006.01)
***A61K 47/10*** (2017.01) ***C12N 15/13*** (2006.01)
***A61K 47/12*** (2006.01) ***C12N 15/56*** (2006.01)
***A61K 47/18*** (2017.01)

(52) Cooperative Patent Classification (CPC):
**A61J 1/05; A61K 9/08; A61K 9/19; A61K 38/02; A61K 38/43; A61K 38/45; A61K 38/46; A61K 38/47; A61K 38/48; A61K 39/395; A61K 45/00; A61K 47/02; A61K 47/10; A61K 47/12; A61K 47/18;** (Cont.)

(86) International application number:
**PCT/JP2022/013755**

(87) International publication number:
**WO 2022/202947 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.03.2021 JP 2021049485**

(71) Applicant: **JCR Pharmaceuticals Co., Ltd.**
**Ashiya-shi, Hyogo 659-0021 (JP)**

(72) Inventors:
- **YASUKAWA, Hidehito**
  **Kobe-shi, Hyogo 651-2241 (JP)**
- **MURASE, Hiroaki**
  **Kobe-shi, Hyogo 651-2241 (JP)**
- **YAMAGUCHI, Yuka**
  **Kobe-shi, Hyogo 651-2241 (JP)**
- **OKABE, Shinji**
  **Kobe-shi, Hyogo 651-2241 (JP)**

(74) Representative: **Boult Wade Tennant LLP**
**Salisbury Square House**
**8 Salisbury Square**
**London EC4Y 8AP (GB)**

Remarks:
The complete document including Reference Table(s) and the Sequence Listing(s) can be downloaded from the EPO website

# (54) STABLE AQUEOUS PHARMACEUTICAL COMPOSITION OR FREEZE-DRIED PHARMACEUTICAL COMPOSITION

(57) Disclosed is a stable aqueous pharmaceutical composition or lyophilized pharmaceutical composition comprising a protein as the active ingredient. The pharmaceutical composition comprises a protein having physiological activity and two different nonionic surfactants, including polysorbate 80 and polyoxyethylene(160) polyoxypropylene(30) glycol as the nonionic surfactants, for example, and as optional components, sodium chloride as a neutral salt, sucrose as a disaccharide and citrate buffer as a buffering agent.

EP 4 311 555 A1

*Fig.7*

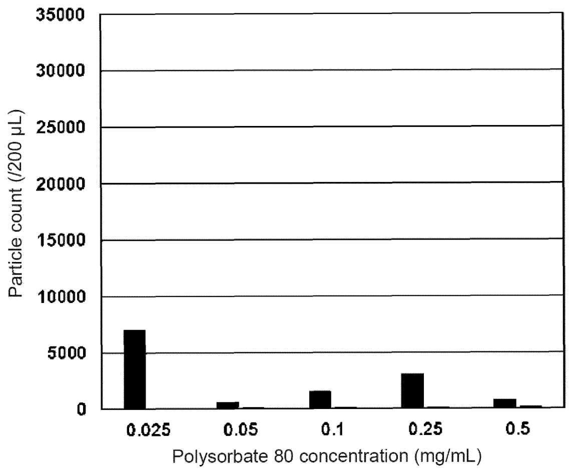


(52) Cooperative Patent Classification (CPC): (Cont.)
**A61K 47/22; A61K 47/26; A61K 47/34; A61K 47/65; A61K 47/68; A61P 3/00; A61P 25/00; A61P 43/00; C07K 16/00; C07K 16/28; C07K 16/46; C12N 9/24; C12N 15/62**

## Description

### Technical Field

The present invention relates to an aqueous pharmaceutical composition comprising a protein having physiological activity, and two or more different nonionic surfactants, and further relates to an aqueous or lyophilized pharmaceutical composition comprising a fusion protein in which an antibody and a lysosomal enzyme are combined, as the protein having physiological activity, and polysorbate 80 and polyoxyethylene(160) polyoxypropylene(30) glycol, as nonionic surfactants.

### Background Art

In the past It was previously common for medicines comprising proteins as active ingredients to be supplied as lyophilized preparations (lyophilized pharmaceutical compositions) in consideration of storage stability of the proteins. Currently, however, most medicines comprising proteins as active compounds, including lysosomal enzymes such as iduronate-2-sulfatase, α-galactosidase A, glucocerebrosidase and α-L-iduronidase-N-acetylgalactosamine-4-sulfatase, antibodies such as anti-human IL-6 receptor antibody and anti-human PD-1 antibody, and erythropoietin, darbepoetin or growth hormones, are produced and marketed in the form of aqueous pharmaceutical compositions. Aqueous pharmaceutical compositions do not require thawing of the medicines at the time of use and are therefore much more convenient than lyophilized pharmaceutical compositions. However, some medicines comprising proteins as active compounds are still provided as lyophilized pharmaceutical compositions.

In order to increase the stability of a protein as the active compound in an aqueous pharmaceutical composition, or to prevent adsorption of the protein onto its container, it is very common to add a nonionic surfactant. Polysorbate 80 is a common example of a nonionic surfactant that is used. For example, some aqueous pharmaceutical compositions comprising darbepoetin or agalsidase as the active ingredient have polysorbate 80 added as a nonionic surfactant (NPLs 1 and 2). Aqueous pharmaceutical compositions of growth hormones may also have polyoxyethylene(160) polyoxypropylene(30) glycol added as a nonionic surfactant (NPL 3).

### Citation List

### Non-Patent Literature

[NPL 1] Darbepoetin alpha BS injection, 5 μg syringe "JCR" (2010)

[NPL 2] Agalsidase beta BS intravenous infusion, 5 mg " JCR" (2018)

[NPL 3] GROWJECT subcutaneous injection, 6 mg/GROWJECT subcutaneous injection, 12 mg, package insert (2017)


### Summary of Invention

### Technical Problem

An objective of the present invention is to provide an aqueous pharmaceutical composition comprising two or more different nonionic surfactants as surfactants and comprising a protein having physiological activity as the active ingredient, which is stable enough for market distribution.

### Solution to Problem

During the course of research conducted with the aforementioned object in mind, the present inventors completed this invention after finding that if a protein having physiological activity is provided in the form of an aqueous pharmaceutical composition or lyophilized pharmaceutical composition comprising sucrose and two different nonionic surfactants as excipients, the composition can be stably stored. Specifically, the present invention provides the following.

1. An aqueous pharmaceutical composition or lyophilized pharmaceutical composition comprising a protein having physiological activity, and two different nonionic surfactants.
2. The aqueous pharmaceutical composition or lyophilized pharmaceutical composition according to 1. above, which further comprises one or more of a neutral salt, a disaccharide and a buffering agent.

3. The aqueous pharmaceutical composition or lyophilized pharmaceutical composition according to 1. or 2. above, which includes a polysorbate and poloxamer as the nonionic surfactants.
4. The aqueous pharmaceutical composition or lyophilized pharmaceutical composition according to 3. above, wherein:

the polysorbate is polysorbate 20 or polysorbate 80, and
the poloxamer is selected from the group consisting of polyoxyethylene(42) polyoxypropylene(67) glycol, polyoxyethylene(54) polyoxypropylene(39) glycol, polyoxyethylene(196) polyoxypropylene(67) glycol, polyoxyethylene(42) polyoxypropylene(67) glycol, polyoxyethylene(3) polyoxypropylene(17) glycol, polyoxyethylene(20) polyoxypropylene(20) glycol and polyoxyethylene (120) polyoxypropylene(40) glycol.

5. The aqueous pharmaceutical composition or lyophilized pharmaceutical composition according to 3. above, wherein the polysorbate is polysorbate 80 and the poloxamer is polyoxyethylene(160) polyoxypropylene(30) glycol.
6. The aqueous pharmaceutical composition according to any one of 3. to 5. above, wherein the concentration of the polysorbate is 0.005 to 1.5 mg/mL and the concentration of the poloxamer is 0.1 to 0.6 mg/mL, or the concentration of the polysorbate is 0.005 to 1.5 mg/mL and the concentration of the poloxamer is 0.05 to 0.6 mg/mL.
7. The aqueous pharmaceutical composition according to any one of 3. to 5. above, wherein the concentration of the polysorbate is 0.025 to 1.0 mg/mL and the concentration of the poloxamer is 0.2 to 0.5 mg/mL, or the concentration of the polysorbate is 0.025 to 1.0 mg/mL and the concentration of the poloxamer is 0.1 to 0.5 mg/mL.
8. The aqueous pharmaceutical composition according to any one of 3. to 5. above, wherein the concentration of the polysorbate is 0.05 to 0.15 mg/mL and the concentration of the poloxamer is 0.25 to 0.45 mg/mL, or the concentration of the polysorbate is 0.05 to 0.15 mg/mL and the concentration of the poloxamer is 0.15 to 0.45 mg/mL.
9. The aqueous pharmaceutical composition according to any one of 1. to 8. above, wherein the neutral salt is sodium chloride.
10. The aqueous pharmaceutical composition according to any one of 1. to 9. above, wherein the disaccharide is selected from the group consisting of trehalose, sucrose, maltose, lactose and combinations of two or more of the foregoing.
11. The aqueous pharmaceutical composition according to any one of 1. to 10. above, wherein the buffering agent is selected from the group consisting of citrate buffer, phosphate buffer, glycine buffer, histidine buffer, carbonate buffer, acetate buffer, and combinations of two or more of the foregoing.
12. The aqueous pharmaceutical composition according to any one of 3. to 5. above, wherein the concentration of the neutral salt is 0.3 to 1.2 mg/mL, the concentration of the disaccharide is 50 to 100 mg/mL, the concentration of the buffering agent is 10 to 30 mM, the concentration of the polysorbate is 0.005 to 1.5 mg/mL and the concentration of the poloxamer is 0.1 to 0.6 mg/mL.
13. The aqueous pharmaceutical composition according to any one of 3. to 5. above, wherein the concentration of the neutral salt is 0.5 to 1.0 mg/mL, the concentration of the disaccharide is 55 to 95 mg/mL, the concentration of the buffering agent is 15 to 25 mM, the concentration of the polysorbate is 0.05 to 1.0 mg/mL and the concentration of the poloxamer is 0.25 to 0.45 mg/mL.
14. The aqueous pharmaceutical composition according to any one of 3. to 5. above, wherein the concentration of the neutral salt is 0.7 to 0.9 mg/mL, the concentration of the disaccharide is 60 to 90 mg/mL, the concentration of the buffering agent is 15 to 25 mM, the concentration of the polysorbate is 0.05 to 0.15 mg/mL and the concentration of the poloxamer is 0.25 to 0.45 mg/mL.
15. The aqueous pharmaceutical composition according to any one of 1. to 14. above, wherein the pH is 4.5 to 6.5.
16. The aqueous pharmaceutical composition according to any one of 1. to 14. above, wherein the pH is 5.0 to 6.0.
17. The aqueous pharmaceutical composition according to any one of 1. to 14. above, wherein the pH is 5.2 to 5.8.
18. The aqueous pharmaceutical composition according to any one of 1. to 17. above, wherein the protein having physiological activity is the fusion protein of an antibody and a lysosomal enzyme.
19. The aqueous pharmaceutical composition according to 18. above, wherein the fusion protein is the lysosomal enzyme bonded by a peptide bond at either the C-terminus or N-terminus of either the antibody light chain or heavy chain.
20. The aqueous pharmaceutical composition according to 18. above, wherein the fusion protein is the lysosomal enzyme bonded by a peptide bond at the C-terminus of the antibody heavy chain.
21. The aqueous pharmaceutical composition according to 18. above, wherein the fusion protein is the lysosomal enzyme bonded at either the C-terminus or N-terminus of either the antibody light chain or heavy chain via a linker consisting of at least one amino acid.
22. The aqueous pharmaceutical composition according to 18. above, wherein the fusion protein is the lysosomal enzyme bonded at the C-terminus of the antibody heavy chain via a linker consisting of at least one amino acid.
23. The aqueous pharmaceutical composition according to 21. or 22. above, wherein the linker has an amino acid sequence selected from the group consisting of Gly-Ser, Gly-Gly-Ser, SEQ ID NO: 1, SEQ ID NO:2, SEQ ID NO: 3, SEQ ID NO: 4, and 1 to 10 of any of aforementioned amino acid sequences that are consecutively linked.
24. The aqueous pharmaceutical composition according to any one of 18. to 23. above, wherein the lysosomal enzyme is a human lysosomal enzyme.
25. The aqueous pharmaceutical composition according to any one of 18. to 24. above, wherein the lysosomal enzyme is selected from the group consisting of α-L-iduronidase, iduronate-2-sulfatase, glucocerebrosidase, β-galactosidase, GM2 activated protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase, β-glucuronidase, heparan N-sulfatase, α-N-acetylglucosaminidase, acetyl CoAα-glucosaminide N-acetyltransferase, N-acetylglucosamine-6-sulfatase, acid ceramidase, amylo-1,6-glucosidase, sialidase, aspartylglucosaminidase, palmitoyl protein thioesterase-1, tripeptidyl peptidase-1, hyaluronidase-1, CLN1 and CLN2.
26. The aqueous pharmaceutical composition according to 24. above, wherein the human lysosomal enzyme is α-L-iduronidase.
27. The aqueous pharmaceutical composition according to any one of 18. to 26. above, wherein the antibody is a human antibody or humanized antibody.
28. The aqueous pharmaceutical composition according to any one of 18. to 27. above, wherein the antibody is a Fab antibody, $F(ab')_2$ antibody or F(ab') antibody.
29. The aqueous pharmaceutical composition according to any one of 18. to 28. above, wherein the antibody recognizes as antigen a molecule present on the surfaces of vascular endothelial cells.
30. The aqueous pharmaceutical composition according to 29. above, wherein the vascular endothelial cells are human vascular endothelial cells.
31. The aqueous pharmaceutical composition according to 29. or 30. above, wherein the vascular endothelial cells are cerebrovascular endothelial cells.
32. The aqueous pharmaceutical composition according to 31. above, wherein the molecule present on the surfaces of cerebrovascular endothelial cells is selected from the group consisting of transferrin receptor (TfR), insulin receptor, leptin receptor, lipoprotein receptor, IGF receptor, OATP-F, organic anion transporter and monocarboxylate transporter.
33. The aqueous pharmaceutical composition according to 28. above, wherein the antibody is a humanized anti-human transferrin receptor (hTfR) antibody.
34. The aqueous pharmaceutical composition according to 28. above, wherein the antibody is the Fab antibody of humanized anti-human transferrin receptor (hTfR) antibody, the human lysosomal enzyme is human α-L-iduronidase, the fusion protein is a fusion protein of the antibody and the human α-L-iduronidase, and in the fusion protein:

(1) the antibody light chain includes the amino acid sequence set forth as SEQ ID NO: 22, and
(2) the antibody heavy chain is bonded at the C-terminus with human α-L-iduronidase via the amino acid sequence set forth as SEQ ID NO: 4, thereby forming the amino acid sequence set forth as SEQ ID NO: 27.

35. The aqueous pharmaceutical composition according to 28. above, wherein the antibody is the Fab antibody of humanized anti-human transferrin receptor (hTfR) antibody, the human lysosomal enzyme is human α-L-iduronidase, the fusion protein is a fusion protein of the antibody and the human α-L-iduronidase, and in the fusion protein:

(1) the antibody light chain includes the amino acid sequence set forth as SEQ ID NO: 22, and
(2) the antibody heavy chain includes the amino acid sequence set forth as SEQ ID NO: 23, the heavy chain being bonded at the C-terminus with human α-L-iduronidase having the amino acid sequence set forth as SEQ ID NO: 5 or SEQ ID NO: 6, via the amino acid sequence set forth as SEQ ID NO: 4.

36. The aqueous pharmaceutical composition according to any one of 1. to 35. above, which is encapsulated in a container formed of borosilicate glass or a hydrophobic resin.
37. The aqueous pharmaceutical composition according to 36. above, wherein the container is formed of a cycloolefin copolymer, a cycloolefin ring-opening polymer or a hydrogenated cycloolefin ring-opening polymer.
38. A pharmaceutical composition that includes a lyophilized aqueous pharmaceutical composition according to any one of 1. to 35. above.
39. The lyophilized pharmaceutical composition according to 38. above, which is encapsulated in a container whose material includes borosilicate glass or a hydrophobic resin.
40. The lyophilized pharmaceutical composition according to 39. above, wherein the material of the container includes a cycloolefin copolymer, a cycloolefin ring-opening polymer or a hydrogenated cycloolefin ring-opening polymer.
41. The aqueous pharmaceutical composition or lyophilized pharmaceutical composition according to any of 1. to 40. above, wherein the content ratio of the polymer after storage for 36 months in a dark environment at a temperature of 2 to 8°C is 0.5% or lower.

42. The aqueous pharmaceutical composition according to any of 1. to 37. above, wherein the content ratio of the polymer and the content ratio of decomposition products after storage for 36 months in a dark environment at a temperature of 2 to 8°C are 0.5% or lower and 1% or lower, respectively.

42. The lyophilized pharmaceutical composition according to any of 39. to 41. above, wherein the content ratio of the polymer and the content ratio of decomposition products after storage for 36 months in a dark environment at a temperature of 2 to 8°C are 0.5% or lower and 0.1% or lower, respectively.

## Advantageous Effects of Invention

The invention enables an aqueous pharmaceutical composition or lyophilized pharmaceutical composition to be provided that comprises a protein having physiological activity as an active ingredient, and that is stable enough for market distribution.

## Brief Description of Drawings

Fig. 1 is a graph showing measured values for the number of particles per unit liquid volume (200 μL) in aqueous pharmaceutical compositions (formulations A to C) after shaking for 24 hours. The black bars represent the number of particles with particle diameters of less than 10 μm, and the white bars represent the number of particles with particle diameters of 10 μm or greater. The ordinate represents number of particles (count/200 μL) and the abscissa represents the concentration of poloxamer 188 (mg/mL).

Fig. 2 is a graph showing polymer contents of humanized anti-hTfR antibody-hIDUA in aqueous pharmaceutical compositions (formulations A to C) after shaking for 24 hours. The ordinate represents the polymer content (%), and the abscissa represents the concentration of poloxamer 188 (mg/mL).

Fig. 3 is a graph showing decomposition product contents of humanized anti-hTfR antibody-hIDUA in aqueous pharmaceutical compositions (formulations A to C) after shaking for 24 hours. The ordinate represents the polymer content (%), and the abscissa represents the concentration of poloxamer 188 (mg/mL).

Fig. 4 is a graph showing measured values for the number of particles per unit liquid volume (200 μL) in aqueous pharmaceutical compositions (formulations D to I) after shaking for 24 hours. The black bars represent the number of particles with particle diameters of less than 10 μm, and the white bars represent the number of particles with particle diameters of 10 μm or greater. The ordinate represents number of particles (count/200 μL) and the abscissa represents the concentration of polysorbate 80 (mg/mL).

Fig. 5 is a graph showing polymer contents of humanized anti-hTfR antibody-hIDUA in aqueous pharmaceutical compositions (formulations D to I) after shaking for 24 hours. The ordinate represents the polymer content (%), and the abscissa represents the concentration of polysorbate 80 (mg/mL).

Fig. 6 is a graph showing decomposition product contents of humanized anti-hTfR antibody-hIDUA in aqueous pharmaceutical compositions (formulations D to I) after shaking for 24 hours. The ordinate represents the polymer content (%), and the abscissa represents the concentration of polysorbate 80 (mg/mL).

Fig. 7 is a graph showing measured values for the number of particles per unit liquid volume (200 μL) in aqueous pharmaceutical compositions (formulations J to N) after shaking for 24 hours. The black bars represent the number of particles with particle diameters of less than 10 μm, and the white bars represent the number of particles with particle diameters of 10 μm or greater. The ordinate represents number of particles (count/200 μL) and the abscissa represents the concentration of polysorbate 80 (mg/mL).

Fig. 8 is a graph showing polymer contents of humanized anti-hTfR antibody-hIDUA in aqueous pharmaceutical compositions (formulations J to N) after shaking for 24 hours. The ordinate represents the polymer content (%), and the abscissa represents the concentration of polysorbate 80 (mg/mL).

Fig. 9 is a graph showing decomposition product contents of humanized anti-hTfR antibody-hIDUA in aqueous pharmaceutical compositions (formulations J to N) after shaking for 24 hours. The ordinate represents the polymer content (%), and the abscissa represents the concentration of polysorbate 80 (mg/mL).

## Description of Embodiments

The present invention relates to a pharmaceutical composition that can be stably stored in a solution or lyophilized state comprising a protein having physiological activity as the active ingredient. The protein having physiological activity may include a protein in which an antibody and a bioactive substance are bonded. The animal species of the antibody to be bonded with the bioactive substance is not particularly limited so long as it has the property of specifically binding to antigen, but a human antibody or humanized antibody is preferred. For example, the antibody may be an antibody of a mammal other than a human, or it may be a chimeric antibody of a human antibody and an antibody of a mammal other than a human.

A human antibody is an antibody that is entirely encoded by a human gene. However, an antibody encoded by a gene having a mutation to an original human gene for the purpose of increasing efficiency of expression of the gene is also included within the definition of "human antibody". An antibody in which two or more genes coding for human antibodies are combined, with part of one human antibody replacing part of another human antibody, is also a human antibody. A human antibody has three complementarity determining regions (CDR) on the immunoglobulin light chain, and three complementarity determining regions (CDR) on the immunoglobulin heavy chain. The three CDRs of the immunoglobulin light chain are designated as CDR1, CDR2 and CDR3, in order from the N-terminus. The three CDRs of the immunoglobulin heavy chain are likewise designated as CDR1, CDR2 and CDR3, in order from the N-terminus. An antibody in which the antigen specificity or affinity of a human antibody is modified by replacing a CDR of one human antibody with a CDR of another human antibody, is also a human antibody.

According to one embodiment of the present invention, an antibody in which a mutation such as a substitution, deletion or addition has been added to the amino acid sequence of the original antibody by modification of the gene of the original human antibody, is also referred to as a "human antibody". When amino acids in the amino acid sequence of an original antibody are substituted with other amino acids, the number of substituted amino acids is preferably 1 to 20, more preferably 1 to 10, even more preferably 1 to 5 and yet more preferably 1 to 3. When amino acids in the amino acid sequence of an original antibody are deleted, the number of deleted amino acids is preferably 1 to 20, more preferably 1 to 10, even more preferably 1 to 5 and yet more preferably 1 to 3. An antibody with a mutation that is a combination of a substitution and a deletion of amino acids is also a human antibody. When amino acids are added, preferably 1 to 20, more preferably 1 to 10, even more preferably 1 to 5 and yet more preferably 1 to 3 amino acids are added within or at the N-terminus or C-terminus of the amino acid sequence of the original antibody. An antibody with a mutation that is a combination of an addition, substitution and deletion of amino acids is also a human antibody. The amino acid sequence of a mutated antibody has preferably identity of not lower than 80%, more preferably identity of not lower than 85%, even more preferably identity of not lower than 90%, yet more preferably identity of not lower than 95%, and even yet more preferably identity of not lower than 98%, to the amino acid sequence of the original antibody. That is, a "human-derived gene" for the purpose of the present invention includes the original human-derived gene, but also genes obtained by adding modifications to the original human-derived gene.

In the present invention, the term "humanized antibody" refers to an antibody wherein the amino acid sequence of part of the variable region (for example, all or part of the CDR) is derived from a mammal other than a human, and the rest of the region is human-derived. For example, the humanized antibody may be an antibody constructed by replacing the three complementarity determining regions (CDR) on the immunoglobulin light chain and the three complementarity determining regions (CDR) on the immunoglobulin heavy chain of the human antibody with CDRs of another mammal. The species of the other mammal from which the CDRs transplanted into the appropriate sites of the human antibody are derived is not particularly limited so long as it is a mammal other than a human, but it is preferably a mouse, rat, rabbit, horse or non-human primate, and preferably a mouse or rat, such as a mouse.

In the present invention, the following explanation applies when the antibody is a human antibody or humanized antibody. The light chains of a human antibody or humanized antibody are the $\lambda$ chain and $\kappa$ chain. The light chains used to form the antibody may be either $\lambda$ chains or $\kappa$ chains. The heavy chains of a human antibody or humanized antibody are the $\gamma$ chain, $\mu$ chain, $\alpha$ chain, $\sigma$ chain and $\varepsilon$ chain, corresponding to IgG, IgM, IgA, IgD and IgE, respectively. The heavy chains composing the antibody may be $\gamma$ chains, $\mu$ chains, $\alpha$ chains, $\sigma$ chains or $\varepsilon$ chains, and are preferably $\gamma$ chains. The $\gamma$ chains as heavy chains of an antibody include the $\gamma$1 chain, $\gamma$2 chain, $\gamma$3 chain and $\gamma$4 chain, corresponding to IgG1, IgG2, IgG3 and IgG4, respectively. When the heavy chains composing the antibody are $\gamma$ chains, the $\gamma$ chains may be $\gamma$1 chains, $\gamma$2 chains, $\gamma$3 chains or $\gamma$4 chains and are preferably $\gamma$1 chains or $\gamma$4 chains. When the antibody is a humanized antibody or human antibody and is IgG, the light chains of the antibody may be either $\lambda$ chains or $\kappa$ chains, and the heavy chains of the antibody may be $\gamma$1 chains, $\gamma$2 chains, $\gamma$3 chains or $\gamma$4 chains, but $\gamma$1 chains and $\gamma$4 chains are preferred. Examples of a preferred mode of the antibody include the antibodies whose light chain is $\kappa$ chain and the heavy chain is $\gamma$1 chain, and whose the light chain is $\lambda$ chain and the heavy chain is $\gamma$1 chain.

In the present invention, the term "chimeric antibody" refers to an antibody wherein two or more different antibody fragments derived from two or more different species are linked.

A chimeric antibody of a human antibody and an antibody of another mammal is an antibody wherein part of a human antibody has been replaced by part of an antibody of a mammal other than a human. The antibody comprises an Fc region, a Fab region and a hinge region, as explained below. Specific examples of such chimeric antibodies include chimeric antibodies wherein the Fc region is derived from a human antibody while the Fab region is derived from an antibody of another mammal. The hinge region may be derived from either the human antibody or the antibody of the other mammal. Conversely, it may be a chimeric antibody wherein the Fc region is derived from another mammal and the Fab region is derived from a human antibody. The hinge region may be derived from either the human antibody or the antibody of the other mammal. The same applies for a humanized antibody.

The antibody may also be considered to comprise a variable region and a constant region. Other specific examples of chimeric antibodies include antibodies wherein the heavy chain constant region ($C_H$) and the light chain constant region ($C_L$) are derived from a human antibody and the heavy chain variable region ($V_H$) and the light chain variable region ($V_L$) are derived from an antibody of another mammal, or conversely, wherein the heavy chain constant region ($C_H$) and the light chain constant region ($C_L$) are derived from an antibody of another mammal and the heavy chain variable region ($V_H$) and light chain variable region ($V_L$) are derived from a human antibody. The species of the other mammal is not particularly restricted so long as it is a mammal other than a human, but it is preferably a mouse, rat, rabbit, horse or non-human primate, and more preferably a mouse. The same applies for a humanized antibody.

A chimeric antibody of a human antibody and a mouse antibody is typically referred to as a "human/mouse chimeric antibody". A human/mouse chimeric antibody may be a chimeric antibody wherein the Fc region is derived from a human antibody and the Fab region is derived from a mouse antibody, or conversely, a chimeric antibody wherein the Fc region is derived from a mouse antibody and the Fab region is derived from a human antibody. The hinge region may be derived from either the human antibody or the mouse antibody. Other specific examples of human/mouse chimeric antibodies include antibodies wherein the heavy chain constant region ($C_H$) and the light chain constant region ($C_L$) are derived from a human antibody and the heavy chain variable region ($V_H$) and the light chain variable region ($V_L$) are derived from a mouse antibody, or conversely, wherein the heavy chain constant region ($C_H$) and the light chain constant region ($C_L$) are derived from a mouse antibody and the heavy chain variable region ($V_H$) and light chain variable region ($V_L$) are derived from a human antibody. The same applies for a humanized antibody.

An antibody has a basic original construction comprising a total of 4 polypeptide chains including two immunoglobulin light chains and two immunoglobulin heavy chains. When referred to "antibody" herein, however, the term includes , in addition to the antibodies having basic structure, antibodies having the following structures:

(1) antibodies comprising a total of two polypeptide chains including one immunoglobulin light chain and one immunoglobulin heavy chain, and he following which are explain in detail hereinafter:
(2) a single-chain antibody having a linker bonded to the C-terminus of the immunoglobulin light chain and the immunoglobulin heavy chain consecutively bonded to its C-terminus,
(3) a single-chain antibody having a linker bonded to the C-terminus of the immunoglobulin heavy chain and the immunoglobulin light chain consecutively bonded to its C-terminus,
(4) a single-chain antibody (scFv) having a linker bonded to the C-terminus of the variable region of the immunoglobulin heavy chain and the variable region of the immunoglobulin light chain bonded to the its C-terminus, and
(5) a single-chain antibody (scFv) having a linker bonded to the C-terminus of the variable region of the immunoglobulin light chain and the variable region of the immunoglobulin heavy chain bonded to its C-terminus. Furthermore:
(6) antibodies consisting of the Fab region and lacking the Fc region from the basic structure of an antibody in the original meaning , and antibodies consisting of the Fab region and all or part of the hinge region (including Fab, F(ab') and $F(ab')_2$), and
(7) single-domain antibodies are also included within the definition of "antibody" in the present invention. Also included in the scope of "antibody" in the present invention is a single-chain antibody scFv, obtained by bonding the light chain variable region and the heavy chain variable region via a linker.

The term "linker" as used herein means, for example, multiple amino acids bonded by peptide bonds into a peptide chain. The linker consisting of such a peptide chain may also be referred to as a "peptide linker". The "linker" may also be referred to as "linker sequence", depending on context of the specification. By bonding the N-terminus of the linker to the C-terminus of another protein by a peptide bond, and bonding the N-terminus of yet another protein to the C-terminus of the linker, it is possible to form a conjugate of the two proteins via the linker.

An antibody having the basic structure including a total of 4 polypeptide chains comprising two light chains and two heavy chains has three complementarity determining regions (CDR) in the light chain variable region ($V_L$) and three complementarity determining regions (CDR) in the heavy chain variable region ($V_H$). The three CDRs of the light chain are designated as CDR1, CDR2 and CDR3, in order from the N-terminus. The three CDRs of the heavy chain are likewise designated as CDR1, CDR2 and CDR3, in order from the N-terminus. However, an antibody wherein all or portions of the CDRs are incomplete or lacking is still an antibody so long as it has the property of specifically binding to a specific antigen. The regions other than the CDRs of the light chain and heavy chain variable regions ($V_L$ and $V_H$) are referred to as framework regions (FR). The FRs include FR1, FR2, FR3 and FR4, in order from the N-terminus. The CDRs and FRs are usually present in the order: FR1, CDR1, FR2, CDR2, FR3, CDR3, FR4, from the N-terminus. The same applies for a heavy chain antibody composed entirely of heavy chains.

According to one embodiment of the present invention, the Fab is a molecule wherein one light chain including a variable region and the $C_L$ region (light chain constant region) and one heavy chain including a variable region and

the $C_H1$ region (heavy chain constant region 1) are bonded by disulfide bonds at their respective cysteine residues. In Fab, the heavy chain may further include part of the hinge region in addition to the variable region and $C_H1$ region (heavy chain constant region 1), in which case the hinge region must lack the cysteine residues that are normally present and responsible for binding the heavy chains of the antibody together. In Fab, the light chain and heavy chain are bonded by disulfide bonds formed between the cysteine residues present in the light chain constant region ($C_L$ region) and the cysteine residues present in the heavy chain constant region ($C_H1$ region) or hinge region. The heavy chain forming the Fab is referred to as the Fab heavy chain. Since Fab lacks cysteine residues that are in the hinge region and responsible for binding the heavy chains of the antibody together, it consists of one light chain and one heavy chain. The light chain forming the Fab includes a variable region and the $C_L$ region. The heavy chain forming Fab may be one consisting of the variable region and the $C_H1$ region, or it may include part of the hinge region in addition to the variable region and the $C_H1$ region. In this case, however, the hinge region is selected so that it contains no cysteine residues that might cause binding between the heavy chains, so that disulfide bonds do not form between the two heavy chains in the hinge region. In F(ab'), the heavy chain includes, in addition to the variable region and the $C_H1$ region, also all or part of a hinge region that includes a cysteine residue that cause binding between the heavy chains. $F(ab')_2$ is a molecule wherein two F(ab') fragments are bonded by disulfide bonds at cysteine residues present in the hinge regions. The heavy chain forming F(ab') or $F(ab')_2$ is referred to as the Fab' heavy chain. A polymer such as a dimer or trimer that consists of multiple antibodies bonded either directly or via a linker is also considered to be an antibody. Without limitation to these, however, the concept of "antibody" in the present invention also encompasses any molecule that includes part of an immunoglobulin molecule and has the property of specifically binding to an antigen. In other words, the term "immunoglobulin light chain" as used herein includes any chain that derives from an immunoglobulin light chain and has part or the entirety of the amino acid sequence of its variable region. Likewise, the term "immunoglobulin heavy chain" as used herein includes any chain that derives from an immunoglobulin heavy chain and has part or the entirety of the amino acid sequence of its variable region. Therefore, a heavy chain that lacks the Fc region, for example, is still an immunoglobulin heavy chain so long as it has all or part of the amino acid sequence of the variable region.

The Fc or Fc region referred to here is a region of the antibody molecule that includes a fragment consisting of the $C_H2$ region (the heavy chain constant region 2) and the $C_H3$ region (the heavy chain constant region 3).

An antibody according to one embodiment of the present invention also includes:

(8) scFab, scF(ab') and $scF(ab')_2$, which are single-chain antibodies obtained by bonding the respective light chains and heavy chains composing Fab, F(ab') or $F(ab')_2$ described in (6) above, via a linker sequence. For scFab, scF(ab') and $scF(ab')_2$, each may be one having a linker sequence bonded at the C-terminus of the light chain, and a heavy chain further bonded to its C-terminus, or one having a linker bonded to the C-terminus of the heavy chain and a light chain further bonded to its C-terminus. Also included in the scope of "antibody" in the present invention is the single-chain antibody scFv, obtained by bonding the light chain variable region and the heavy chain variable region via a linker. For scFv, it may be one having a linker sequence bonded to the C-terminus of a light chain variable region and a heavy chain variable region further bonded to its C-terminus, or it may be one having a linker sequence bonded to the C-terminus of a heavy chain variable region and a light chain variable region further bonded to its C-terminus.

The term "antibody" as used herein also includes, in addition to full length antibodies and the antibodies as described by (1) to (8) above, also any form of antigen-binding fragment (antibody fragment) lacking part of the full length antibodies, the antibody fragment having a wider concept that includes (1) to (8). Antigen-binding fragments include heavy chain antibodies, light chain antibodies, VHH and VNAR, as well as those lacking portions thereof.

The term "antigen-binding fragment" is a fragment of an antibody that retains at least part of the specific binding activity with antigen. Examples of binding fragments also include, in addition to those described by (4) and (5) above, Fab, Fab', $F(ab')_2$, variable regions (Fv), a single-chain antibody (scFv) having a heavy chain variable region ($V_H$) and light chain variable region ($V_L$) linked with an appropriate linker, a diabody that is a dimer of polypeptide including a heavy chain variable region ($V_H$) and a light chain variable region ($V_L$), a minibody that is a dimer of a heavy chain (H chain) of a scFv bonded with a part of a constant region ($C_H3$), and any of other low molecular weight antibodies. However, it is not limited to these, but so long as the fragment has the ability to bind with antigen. Such binding fragments include antibodies treated with an appropriate enzyme, and proteins produced in suitable host cells using a genetically modified antibody gene.

According to one embodiment of the present invention, "single-chain antibody" refers to a protein that has a linker bonded at the C-terminus of an amino acid sequence including all or part of the variable region of an immunoglobulin light chain, and further has an amino acid sequence including all or part of the variable region of an immunoglobulin heavy chain bonded to its C-terminus, and that is able to bind specifically to a specific antigen. Moreover, a protein that has a linker bonded at the C-terminus of an amino acid sequence including all or part of the variable region of an immunoglobulin heavy chain, and further has an amino acid sequence including all or part of the variable region of an immunoglobulin light chain bonded to its C-terminus, and that is able to bind specifically to a specific antigen, is also "single-chain antibody" in the present invention. For example, the antibodies described by (2) and (3) above are included in the definition of "single-chain antibody". In a single-chain antibody having an immunoglobulin light chain bonded to the C-terminus of an immunoglobulin heavy chain via a linker, the Fc region of the immunoglobulin heavy chain usually is deleted. The variable region of the immunoglobulin light chain has three complementarity determining regions (CDR) that contribute to the antigen specificity of the antibody. The variable region of the immunoglobulin heavy chain likewise has three CDRs. The CDRs are the main regions that determine the antigen specificity of the antibody. A single-chain antibody therefore preferably includes all of the three CDRs of the immunoglobulin heavy chain and all of the three CDRs of the immunoglobulin light chain. However, the single-chain antibody may lack one or more CDRs so long as the antigen-specific affinity of the antibody is maintained.

In a single-chain antibody, the linker situated between the light chain and heavy chain of an immunoglobulin is a peptide chain composed of amino acid residues in a number of preferably 2 to 50, more preferably 8 to 50, even more preferably 10 to 30 and yet preferably 30 to 30 or 30 to 30, such as either 30 or 30. There is no limitation as to the amino acid sequence of such a linker,, so long as an anti-hTfR antibody having both chains linked by it still retains affinity with hTfR, but it is preferably composed of glycine alone or of glycine and serine, having the amino acid sequence Gly-Ser, the amino acid sequence Gly-Gly-Ser, the amino acid sequence Gly-Gly-Gly, the amino acid sequence set forth as SEQ ID NO: 1, the amino acid sequence set forth as SEQ ID NO: 2, the amino acid sequence set forth as SEQ ID NO: 3, the amino acid sequence set forth as SEQ ID NO: 4, or a sequence with these aforementioned amino acid sequences repeated 2 to 10 times or 2 to 5 times. For example, when the variable region of an immunoglobulin light chain is bonded via a linker to the C-terminus of the amino acid sequence consisting of the entire region of the variable region of the immunoglobulin heavy chain, to obtain ScFV, a linker having the amino acid sequence set forth as SEQ ID NO: 4 is preferably used.

According to one embodiment of the present invention, the antibody is an antibody derived from an animal belonging to the family Camelidae (which includes alpacas). Some antibodies of Camelidae animals have two heavy chains linked by disulfide bonds. Such antibodies having two heavy chains are referred to as heavy chain antibodies. VHH is an antibody consisting of one heavy chain composing a heavy chain antibody and including the heavy chain variable region , or an antibody consisting of one heavy chain composing a heavy chain antibody but lacking the constant region (CH). VHH is also included in the antibodies according to this embodiment of the present invention. A Camelidae animal-derived antibody (such as VHH) having a mutation added to the amino acid sequence of the Camelidae animal antibody to reduce antigenicity when the antibody is administered to a human, is also an antibody according to one embodiment of the invention. When mutations are to be added to amino acids in a Camelidae animal antibody, the same types of mutations may be added as for other antibodies described herein. An antibody consisting of two light chains linked by disulfide bonds is also an antibody according to this embodiment of the invention. Such antibodies having two light chains are referred to as light chain antibodies.

The antibody according to one embodiment of the present invention is a shark-derived antibody. Shark antibodies have two heavy chains linked by disulfide bonds. Such antibodies having two heavy chains are referred to as heavy chain antibodies. VNAR is an antibody consisting of one heavy chain composing a heavy chain antibody and including the heavy chain variable region, or an antibody consisting of one heavy chain composing a heavy chain antibody but lacking the constant region (CH). VNAR is also included in the antibodies according to this embodiment of the present invention. A shark-derived antibody (such as VNAR) having a mutation added to the amino acid sequence of the shark antibody to reduce antigenicity when the antibody is administered to a human, is also an antibody according to one embodiment of the invention. When mutations are to be added to amino acids in a shark antibody, the same types of mutations may be added as for other antibodies described herein. Humanized shark antibodies are also a type of antibody according to this embodiment.

According to one embodiment, a single-domain antibody is an antibody having the property of specifically binding to antigen at a single variable region. Single-domain antibodies include antibodies wherein the variable region consists entirely of a heavy chain variable region (heavy chain single-domain antibody) and antibodies wherein the variable region consists entirely of a light chain variable region (light chain single-domain antibody). VHH and VNAR are types of single-domain antibodies.

In the present invention, the antigen specifically recognized by the antibody is a molecule present on the surface (surface antigen) of vascular endothelial cells, for example. Such surface antigens include, but are not limited to, transferrin receptor (TfR), insulin receptor, leptin receptor, lipoprotein receptor, IGF receptor, organic anion transporters such as OATP-F, monocarboxylate transporter and Fc receptor. Monocarboxylate transporter (MCT) may also be any of the 14 different subtypes (MCT1 to MCT14), but MCT1, MCT2, MCT4 or MCT8 is preferred, such as MCT8. The antigen is preferably a molecule present on the surface (surface antigen) of human vascular endothelial cells.

Among the surface antigens mentioned above, transferrin receptor (TfR), insulin receptor, leptin receptor, lipoprotein receptor, IGF receptor, OATP-F, organic anion transporters such as OATP-F and monocarboxylate transporters such as MCT-8 are present on the surfaces of brain capillary endothelial cells (cerebrovascular endothelial cells) forming the blood brain barrier. Antibodies that can recognize these antigens are able to bind to brain capillary endothelial cells via the antigens. Once the antibody has bound to brain capillary endothelial cells, then it can reach the central nervous system through the blood brain barrier. It is therefore possible to let a target protein reach the central nervous system by binding with such an antibody. The target protein may be a protein that functions to exhibit a drug effect in the central nervous system. One example of a target protein is a lysosomal enzyme that is deficient or dysfunctional in a patient with lysosomal disease associated with central nerve damage. Such a lysosomal enzyme cannot reach the central nervous system by itself and therefore does not exhibit a drug effect against central nerve damage in the patient, but by bonding with the antibody it can pass through the blood brain barrier, thus allowing amelioration of central nerve damage in a lysosomal disease patient.

In the present invention, "human transferrin receptor" or "hTfR" refers to a membrane protein having the amino acid sequence set forth as SEQ ID NO: 5. According to one embodiment, the anti-hTfR antibody of the invention is one that specifically binds to the portion of the amino acid sequence set forth as SEQ ID NO: 5 from the cysteine residue at position 89 from the N-terminus to phenylalanine at the C-terminus (the extracellular domain of hTfR), but this is not limitative.

The antibody creation method will now be described, using antibody for hTfR as an example. The method for creating the antibody for hTfR will generally be a method of using cells transfected with an expression vector incorporating the hTfR gene to produce recombinant human transferrin receptor (rhTfR), and using the rhTfR for immunization of an animal such as a mouse. By taking the cells producing antibody for hTfR from the immunized animals and fusing them with myeloma cells, it is possible to create hybridoma cells having the ability to produce antibodies for hTfR.

Cells producing antibodies for hTfR can also be obtained by immunizing immune system cells obtained from an animal such as a mouse, with rhTfR by an *in vitro* immunization method. For *in vitro* immunization, the animal species from which the immune system cells are derived is not particularly limited, but it is preferably a mouse, rat, rabbit, guinea pig, dog, cat or horse, or a primate such as human, more preferably a mouse, rat or human, and even more preferably a mouse or human. Mouse immune system cells may be splenocytes prepared from mouse spleen, for example. Human immune system cells may be cells from human peripheral blood, bone marrow or spleen. Human antibodies for hTfR can be obtained by immunizing human immune system cells by *in vitro* immunization.

In the present invention there are no particular restrictions on the human lysosomal enzyme to be bonded with the antibody, and it may be a lysosomal enzyme selected from among α-L-iduronidase, iduronate-2-sulfatase, glucocerebrosidase, β-galactosidase, GM2 activated protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl CoAα-glucosaminide N-acetyltransferase, N-acetylglucosamine-6-sulfatase, acid ceramidase, amylo-1,6-glucosidase, sialidase, aspartylglucosaminidase, palmitoyl protein thioesterase-1 (PPT-1), tripeptidyl peptidase-1 (TPP-1), hyaluronidase-1, CLN1, CLN2, CLN3, CLN6 and CLN8.

If the antibody is one that specifically recognizes a molecule present on the surfaces (surface antigen) of vascular endothelial cells, then when the human lysosomal enzyme combined with the antibody is α-L-iduronidase it may be used as a central nervous system disorder therapeutic agent for Hurler syndrome, Hurler-Scheie syndrome or Scheie's syndrome, when it is iduronate-2-sulfatase it may be used as a central nervous system disorder therapeutic agent for Hunter's syndrome, when it is glucocerebrosidase it may be used as a central nervous system disorder therapeutic agent for Gaucher disease, when it is β-galactosidase it may be used as a central nervous system disorder therapeutic agent for GM1-gangliosidosis type 1 to 3, when it is a GM2 activated protein it may be used as a central nervous system disorder therapeutic agent for GM2-gangliosidosis AB variant, when it is β-hexosaminidase A it may be used as a central nervous system disorder therapeutic agent for Sandhoffs disease and Tay-Sachs disease, when it is β-hexosaminidase B it may be used as a central nervous system disorder therapeutic agent for Sandhoffs disease, when it is N-acetylglucosamine-1-phosphotransferase it may be used as a central nervous system disorder therapeutic agent for I-cell disease, when it is α-mannosidase it may be used as a central nervous system disorder therapeutic agent for alpha-mannosidosis, when it is β-mannosidase it may be used as a central nervous system disorder therapeutic agent for beta-mannosidosis, when it is galactosylceramidase it may be used as a central nervous system disorder therapeutic agent for Krabbe disease, when it is saposin C it may be used as a central nervous system disorder therapeutic agent for Gaucher-like storage disease, when it is arylsulfatase A it may be used as a central nervous system disorder therapeutic agent for metachromatic white matter degeneration (metachromatic leukodystrophy), when it is α-L-fucosidase it may be used as a central nervous system disorder therapeutic agent for fucosidosis, when it is aspartylglucosaminidase it may be used as a central nervous system disorder therapeutic agent for aspartylglucosaminuria, when it is α-N-acetylgalactosaminidase it may be used as a central nervous system disorder therapeutic agent for Schindler's disease and Kawasaki disease, when it is acid sphingomyelinase it may be used as a central nervous system disorder therapeutic agent for Niemann-Pick disease, when it is α-galactosidase A it may be used as a central nervous system disorder therapeutic agent for Fabry disease, when it is β-glucuronidase it may be used as a central nervous system disorder therapeutic agent for Sly syndrome, when it is heparan N-sulfatase, α-N-acetylglucosaminidase, acetyl CoA α-glucosaminide N-acetyltransferase or N-acetylglucosamine-6-sulfatase it may be used as a central nervous system disorder therapeutic agent for Sanfilippo syndrome, when it is acid ceramidase it may be used as a central nervous system disorder therapeutic agent for Farber's disease, when it is amylo-1,6-glucosidase it may be used as a central nervous system disorder therapeutic agent for Cori disease (Forbes-Cori disease), when it is sialidase it may be used as a central nervous system disorder therapeutic agent for sialidase deficiency, when it is aspartylglucosaminidase it may be used as a central nervous system disorder therapeutic agent for aspartylglucosaminuria, when it is palmitoyl protein thioesterase-1 (PPT-1) it may be used as a central nervous system disorder therapeutic agent for neuronal ceroid lipofuscinosis or Santavuori-Haltia disease, when it is tripeptidyl peptidase-1 (TPP-1) it may be used as a central nervous system disorder therapeutic agent for neuronal ceroid lipofuscinosis or Jansky-Bielschowsky disease, when it is hyaluronidase-1 it may be used as a central nervous system disorder therapeutic agent for hyaluronidase deficiency, and when it is CLN1, CLN2, CLN3, CLN6 or CLN8 it may be used as a central nervous system disorder therapeutic agent for Batten's disease.

When the antibody specifically recognizes a molecule on the surfaces (surface antigen) of vascular endothelial cells, human α-L-iduronidase (hIDUA) is a suitable lysosomal enzyme to be bonded to the antibody. The enzyme hIDUA is a type of lysosomal enzyme having activity which hydrolyzes iduronic acid bonds present in glycosaminoglycan (GAG) molecules such as heparan sulfate and dermatan sulfate. Mucopolysaccharidosis type I is a genetic disease caused by a mutation in the gene encoding this enzyme. Mucopolysaccharidosis type I is classified into Hurler syndrome, Hurler-Scheie syndrome and Scheie's syndrome, with Hurler syndrome being the severe type, Hurler-Scheie syndrome being the intermediate type, and Scheie's syndrome being the mild type. In such patients, heparan sulfate and dermatan sulfate accumulate in tissues, resulting in various symptoms such as corneal clouding and mental retardation. Mental development delay is often not observed in the mild type, however. Since a fusion protein of the antibody with hIDUA passing through the BBB can decompose GAG accumulated in brain tissue, it can be used as a central nervous system disorder therapeutic agent, by being administered to a patient with Hurler syndrome exhibiting mental retardation.

In the present invention, the term "human α-L-iduronidase" or "hIDUA" refers particularly to hIDUA having an amino acid sequence identical to wild type hIDUA. Wild type hIDUA has an amino acid sequence composed of the 628 amino acids set forth as SEQ ID NO: 6. Variants of hIDUA having an amino acid sequence composed of the 626 amino acids set forth as SEQ ID NO: 7 are also encompassed by the term "hIDUA". This is not limitative, however, and so long as the hIDUA exhibits IDUA activity, it may have a mutation such as a substitution, deletion or addition in the amino acid sequence of wild type hIDUA. When amino acids of the amino acid sequence of hIDUA are substituted with other amino acids, the number of substituted amino acids is preferably 1 to 10, more preferably 1 to 5, even more preferably 1 to 3 and yet more preferably 1 to 2. When amino acids of the amino acid sequence of hIDUA are deleted, the number of deleted amino acids is preferably 1 to 10, more preferably 1 to 5, even more preferably 1 to 3 and yet more preferably 1 to 2. Mutations that are combinations of substitutions and deletions of amino acids may also be added. When amino acids are added to hIDUA, preferably 1 to 10, more preferably 1 to 5, even more preferably 1 to 3 and yet more preferably 1 to 2 amino acids are added within or at the N-terminus or C-terminus of the amino acid sequence of hIDUA. Mutations that are combinations of additions, substitutions and deletions of amino acids may also be added. The amino acid sequence of a mutated hIDUA has preferably identity of not lower than 80%, more preferably identity of not lower than 85%, even more preferably identity of not lower than 90%, yet more preferably identity of not lower than 95%, and even yet more preferably identity of not lower than 99%, to the amino acid sequence of the original hIDUA.

Identity between the amino acid sequence of the original protein (including the antibody) and the amino acid sequence of the mutated protein, for the purpose of the present invention, can be easily calculated using a well-known identity calculation algorithm. Examples of such algorithms include BLAST (Altschul SF. J Mol. Biol. 215. 403-10, (1990)), the similarity search method of Pearson and Lipman (Proc. Natl. Acad. Sci. USA. 85. 2444 (1988)), and the local identity algorithm of Smith and Waterman (Adv. Appl. Math. 2. 482-9(1981)).

Substitution of amino acids in the amino acid sequence of the original protein (including the antibody) with other amino acids occur, for example, in an amino acid family including amino acids relevant each other to their side chains and chemical properties. Examples of such amino acid families include the following:

(1) the acidic amino acids aspartic acid and glutamic acid,
(2) the basic amino acids histidine, lysine and arginine,
(3) the aromatic amino acids phenylalanine, tyrosine and tryptophan,
(4) the hydroxy amino acids serine and threonine,
(5) the hydrophobic amino acids methionine, alanine, valine, leucine and isoleucine,
(6) the neutral hydrophilic amino acids cysteine, serine, threonine, asparagine and glutamine,
(7) amino acids that affect orientation on the peptide chain, such as glycine and proline,
(8) the amide-type amino acids asparagine and glutamine, and
(9) amino acids with small side chains, such as alanine and glycine.

That hIDUA has IDUA activity, in the present invention, means that when the hIDUA has been fused with an antibody to produce a fusion protein, it exhibits at least 3% activity with respect to the original activity of wild-type hIDUA. However, the activity is preferably at least 10%, more preferably at least 20%, even more preferably at least 50% and yet more preferably at least 80% with respect to the original activity of wild-type hIDUA. This also applies when the hIDUA fused with the antibody is a mutated form. The antibody is anti-hTfR antibody, for example.

In the present invention, the term "fusion protein" refers to a substance in which an antibody and a human lysosomal enzyme are bonded either directly or via a non-peptide linker or peptide linker. The method for bonding the antibody and human lysosomal enzyme is described in detail below.

The method for bonding the antibody and human lysosomal enzyme is a method of bonding via a non-peptide linker or peptide linker. Non-peptide linkers to be used include polyethylene glycol, polypropylene glycol, copolymers of ethylene glycol and propylene glycol, polyoxyethylated polyols, polyvinyl alcohol, polysaccharides, dextran, polyvinyl ether, biodegradable polymers, lipid polymers, chitins and hyaluronic acid, as well as their derivatives, and combinations of the foregoing. A peptide linker is a peptide chain composed of 1 to 50 peptide bonded amino acids, or a derivative thereof, and whose N-terminal and C-terminal bind to either the antibody or the human lysosomal enzyme to let the antibody and the human lysosomal enzyme covalently are conjugated.

When biotin-streptavidin is used as a non-peptide linker, the antibody is bonded with biotin and the human lysosomal enzyme is bonded with streptavidin, linking together the antibody and human lysosomal enzyme through the biotin and streptavidin bonds, or conversely, the antibody is bonded to streptavidin and the human lysosomal enzyme is bonded to biotin, linking together the antibody and human lysosomal enzyme via the biotin and streptavidin bonds.

A combination of antibody and human lysosomal enzyme of the present invention that are linked using PEG as the non-peptide linker is referred to as an antibody-PEG-human lysosomal enzyme. An antibody-PEG-human lysosomal enzyme can be produced by bonding together an antibody and PEG as antibody-PEG, and then bonding human lysosomal enzyme with the antibody-PEG. Alternatively, an antibody-PEG-human lysosomal enzyme can be produced by bonding together a human lysosomal enzyme and PEG as human lysosomal enzyme-PEG, and then bonding the human lysosomal enzyme-PEG with an antibody. When PEG is used to bond an antibody and human lysosomal enzyme, PEG may be modified with a functional group such as carbonate, carbonylimidazole, an active ester of carboxylic acid, azlactone, cyclic imide thione, isocyanate, isothiocyanate, imidate or aldehyde. A functional group introduced into PEG reacts mainly with amino groups in the antibody or human lysosomal enzyme molecule, allowing covalent bonding between PEG and the antibody and human lysosomal enzyme. The molecular weight and form of the PEG used is not particularly restricted, but the average molecular weight (MW) is preferably MW = 300 to 60,000 and more preferably MW = 500 to 20,000. For example, PEG having an average molecular weight of about 300, about 500, about 1000, about 2000, about 4000, about 10,000 or about 20,000 is suitable for use as a non-peptide linker.

The antibody-PEG can be obtained, for example, by mixing an antibody with polyethylene glycol (ALD-PEG-ALD) having aldehyde groups as functional groups, at an ALD-PEG-ALD to antibody molar ratio of 1:11, 1:12.5, 1:15, 1:110 or 1:120, and adding a reducing agent such as $NaCNBH_3$ for reaction. The antibody-PEG may then be reacted with the human lysosomal enzyme in the presence of a reducing agent such as $NaCNBH_3$ to obtain antibody-PEG-human lysosomal enzyme. Conversely, an antibody-PEG-human lysosomal enzyme can be obtained by bonding together a human lysosomal enzyme and ALD-PEG-ALD as human lysosomal enzyme-PEG, and then bonding the human lysosomal enzyme-PEG with an antibody.

The antibody and the human lysosomal enzyme may have the N-terminus or C-terminus of the human lysosomal enzyme peptide bonded to the C-terminus or N-terminus of the antibody heavy chain or light chain, either directly or via a linker. A fusion protein having an antibody and human lysosomal enzyme linked in this manner can be obtained by incorporating a DNA fragment having cDNA encoding a human lysosomal enzyme situated inframe at the 3'-end or 5'-end of cDNA encoding the antibody heavy chain or light chain, either directly or sandwiching a DNA fragment encoding a linker, into an expression vector for eukaryotes such as mammalian cells or yeast, and culturing mammalian cells transfected with the expression vector. In mammalian cells, for bonding of a DNA fragment encoding a human lysosomal enzyme to a heavy chain, an expression vector for mammalian cells incorporating the cDNA fragment encoding the antibody light chain is introduced into the same host cells, or for bonding of a DNA fragment encoding a human lysosomal enzyme to a light chain, an expression vector for mammalian cells incorporating a cDNA fragment encoding the antibody heavy chain is introduced into the same host cells. When the antibody is a single-chain antibody, the fusion protein having an antibody and human lysosomal enzyme linked together can be obtained by incorporating a DNA fragment in which cDNA encoding a single-chain antibody is connected, on the 5'-terminal side or the 3'-terminal side, to cDNA encoding the human lysosomal enzyme, directly or via the DNA fragment encoding the linker sequence, into the expression vector for Eukaryote such as a mammalian cell and a yeast, and expressing it in such cells to which the expression vector is introduced.

In the fusion protein of a type combining a human lysosomal enzyme at the C-terminus of the light chain of an antibody, the antibody comprises an amino acid sequence including all or part of the light chain variable region and an amino acid sequence including all or part of the heavy chain variable region, with the human lysosomal enzyme being bonded to the C-terminus of the antibody light chain. The antibody light chain and the human lysosomal enzyme may be directly bonded, or they may be linked via a linker.

In the fusion protein of a type combining a human lysosomal enzyme at the C-terminus of the heavy chain of an antibody, the antibody comprises an amino acid sequence including all or part of the light chain variable region and an amino acid sequence including all or part of the heavy chain variable region, with the human lysosomal enzyme being bonded to the C-terminus of the antibody heavy chain. The antibody heavy chain and the human lysosomal enzyme may be directly bonded, or they may be linked via a linker.

In the fusion protein of a type combining a human lysosomal enzyme at the N-terminus of the light chain of an antibody, the antibody comprises an amino acid sequence including all or part of the light chain variable region and an amino acid sequence including all or part of the heavy chain variable region, with the human lysosomal enzyme being bonded to the N-terminus of the antibody light chain. The antibody light chain and the human lysosomal enzyme may be directly bonded, or they may be linked via a linker.

In the fusion protein of a type combining a human lysosomal enzyme at the N-terminus of the heavy chain of an antibody, the antibody comprises an amino acid sequence including all or part of the light chain variable region and an amino acid sequence including all or part of the heavy chain variable region, with the human lysosomal enzyme being bonded to the N-terminus of the antibody heavy chain. The antibody heavy chain and the human lysosomal enzyme may be directly bonded, or they may be linked via a linker.

When a linker is situated between the antibody and the human lysosomal enzyme, its sequence is composed of preferably 1 to 50, more preferably 1 to 17, even more preferably 1 to 10 and yet more preferably 1 to 5 amino acids, but the number of amino acids composing the linker may be, for example, appropriately adjusted to 1, 2, 3, 1 to 17, 1 to 10, 10 to 40, 20 to 34, 23 to 31 or 25 to 29, depending on the human lysosomal enzyme to be bonded to the antibody. There is no limitation to the amino acid sequence of such a linker, so long as the antibody linked by it retains affinity with hTfR and the human lysosomal enzyme linked by the linker can exhibit the physiological activity of the protein under physiological conditions, but it is preferably composed of glycine and serine, such as, for example, a linker composed of a single amino acid which is either glycine or serine, the amino acid sequence Gly-Ser, the amino acid sequence Gly-Gly-Ser, the amino acid sequence set forth as SEQ ID NO: 1, the amino acid sequence set forth as SEQ ID NO: 2, the amino acid sequence set forth as SEQ ID NO: 3, the amino acid sequence set forth as SEQ ID NO: 4, or a sequence comprising 1 to 50 amino acids, or a sequence comprising 2 to 17, 2 to 10, 10 to 40, 20 to 34, 23 to 31 or 25 to 29 amino acids, with the aforementioned amino acid sequences repeated 1 to 10 times or 2 to 5 times. For example, suitable linkers include one having the amino acid sequence Gly-Ser, or one having the amino acid sequence set forth as SEQ ID NO: 4. The same applies when the antibody is a single-chain antibody.

When the fusion protein includes multiple linkers in a single peptide chain, the linkers are designated such as "first linker", "second linker" in order from the N-terminus.

Preferred embodiments of the antibody, when the antibody is an anti-human transferrin receptor antibody, are antibodies wherein in the light chain variable region:

(a) CDR1 includes the amino acid sequence set forth as SEQ ID NO: 8 or 9,
(b) CDR2 includes the amino acid sequence set forth as SEQ ID NO: 10 or 11, and
(c) CDR3 includes the amino acid sequence set forth as SEQ ID NO: 12; and
in the heavy chain variable region,
(d) CDR1 includes the amino acid sequence set forth as SEQ ID NO: 13 or 14,
(e) CDR2 includes the amino acid sequence set forth as SEQ ID NO: 15 or 16, and
(f) CDR3 includes the amino acid sequence set forth as SEQ ID NO: 17 or 18. The antibody is preferably a human antibody or humanized antibody.

The combination of amino acid sequences for each CDR of (a) to (f) above may be any combination, for examples, such as those listed in Table 1.

[Table 1]

| (Table 1) Light and heavy chain CDR amino acid sequence combination example 1 | | | | | | |
|---|---|---|---|---|---|---|
| | Light chain | | | Heavy chain | | |
| | CDR1 | CDR2 | CDR3 | CDR1 | CDR2 | CDR3 |
| SEQ ID NO: | 8 | 10 | 12 | 13 | 15 | 17 |
| | 8 | 10 | 12 | 14 | 16 | 18 |
| | 9 | 11 | 12 | 13 | 15 | 17 |
| | 9 | 11 | 12 | 14 | 16 | 18 |

When the antibody is an anti-human transferrin receptor antibody, preferred embodiments of the antibody also include those wherein:

(x) the light chain variable region has the amino acid sequence set forth as SEQ ID NO: 20, and the heavy chain variable region includes the amino acid sequence set forth as SEQ ID NO: 21. The antibody is preferably a human antibody or humanized antibody.

The amino acid sequence of the light chain variable region set forth as SEQ ID NO: 20 includes the amino acid sequences set forth as SEQ ID NO: 8 and SEQ ID NO: 9 as CDR1, the amino acid sequences set forth as SEQ ID NO: 10 and SEQ ID NO: 11 as CDR2, and the amino acid sequence set forth as SEQ ID NO: 12 as CDR3. The amino acid sequence of the heavy chain variable region set forth as SEQ ID NO: 21 includes the amino acid sequences set forth as SEQ ID NO: 13 and SEQ ID NO: 14 as CDR1, the amino acid sequences set forth as SEQ ID NO: 15 and SEQ ID NO: 16 as CDR2, and the amino acid sequences set forth as SEQ ID NO: 17 and SEQ ID NO: 18 as CDR3. It further includes the amino acid sequence set forth as SEQ ID NO: 19 as the heavy chain framework region 3.

However, preferred embodiments where the antibody is a humanized antibody and is an anti-human transferrin receptor antibody are not limited to (x). For example, an antibody wherein the amino acid sequence of the light chain variable region has identity of not lower than 80% to the amino acid sequence of the light chain variable region of (x) above, and wherein the amino acid sequence of the heavy chain variable region has identity of not lower than 80% to the amino acid sequence of the heavy chain variable region of (x) above, may also be used for the present invention so long as it has affinity for hTfR.

Those described below may also be used for the invention:

an antibody wherein the amino acid sequence of the light chain variable region has identity of not lower than 85% to the amino acid sequence of the light chain variable region of (x) above, and the amino acid sequence of the heavy chain variable region has identity of not lower than 85% to the amino acid sequence of the heavy chain variable region of (x) above,

an antibody wherein the amino acid sequence of the light chain variable region has identity of not lower than 90% to the amino acid sequence of the light chain variable region of (x) above, and the amino acid sequence of the heavy chain variable region has identity of not lower than 90% to the amino acid sequence of the heavy chain variable region of (x) above, and

an antibody wherein the amino acid sequence of the light chain variable region has identity of not lower than 95% to the amino acid sequence of the light chain variable region of (x) above, and the amino acid sequence of the heavy chain variable region has identity of not lower than 95% to the amino acid sequence of the heavy chain variable region of (x) above,

so long as the antibody still has affinity with hTfR.

Antibodies wherein the amino acid sequence of the light chain variable region has identity to the amino acid sequence of the light chain variable region of (x) above and the amino acid sequence of the heavy chain variable region has identity to the amino acid sequence of the heavy chain variable region of (x) above, include the following:

(x-a) an antibody wherein the light chain variable region includes an amino acid sequence having sequence identity of not less than 80% to the amino acid sequence set forth as SEQ ID NO: 20, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain variable region includes an amino acid sequence having sequence identity of not less than 80% to the amino acid sequence of SEQ ID NO: 21, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,

(x-b) an antibody wherein the light chain variable region includes an amino acid sequence having sequence identity of not less than 85% to the amino acid sequence set forth as SEQ ID NO: 20, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain variable region includes an amino acid sequence having sequence identity of not less than 85% to the amino acid sequence set forth as SEQ ID NO: 21, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,

(x-c) an antibody wherein the light chain variable region includes an amino acid sequence having sequence identity of not less than 90% to the amino acid sequence set forth as SEQ ID NO: 20, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain variable region includes an amino acid sequence having sequence identity of not less than 90% to the amino acid sequence set forth as SEQ ID NO: 21, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,

(x-d) an antibody wherein the light chain variable region includes an amino acid sequence having sequence identity of not less than 95% to the amino acid sequence set forth as SEQ ID NO: 20, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain variable region includes an amino acid sequence having sequence identity of not less than 95% to the amino acid sequence set forth as SEQ ID NO: 21, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3.

When the antibody is an anti-human transferrin receptor antibody, preferred embodiments of the antibody to be used for the invention include:

an antibody having the amino acid sequence of the light chain variable region of (x) above with a substitution, deletion or addition of 1 to 5 amino acids in the constituent amino acid sequence, and the amino acid sequence of the heavy chain variable region of (x) above with a substitution, deletion or addition of 1 to 5 amino acids in the constituent amino acid sequence,
an antibody having the amino acid sequence of the light chain variable region of (x) above with a substitution, deletion or addition of 1 to 3 amino acids in the constituent amino acid sequence, and the amino acid sequence of the heavy chain variable region of (x) above with a substitution, deletion or addition of 1 to 3 amino acids in the constituent amino acid sequence, and
an antibody having the amino acid sequence of the light chain variable region of (x) above with a substitution, deletion or addition of 1 or 2 amino acids in the constituent amino acid sequence, and the amino acid sequence of the heavy chain variable region of (x) above with a substitution, deletion or addition of 1 or 2 amino acids in the constituent amino acid sequence,

so long as the antibody still has affinity for hTfR. The antibody is preferably a human antibody or humanized antibody.
Antibodies wherein the amino acid sequence of the light chain variable region of the antibody has the amino acid sequence of the light chain variable region of (x) above with a substitution, deletion or addition of an amino acid in the constituent amino acid sequence and the amino acid sequence of the heavy chain variable region of (x) above with a substitution, deletion or addition of an amino acid in the constituent amino acid sequence, include the following:

(x-e)
an antibody having the amino acid sequence of the light chain variable region with a substitution, deletion or addition of 1 to 5 amino acids in the constituent amino acid sequence, which includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and having the amino acid sequence of the heavy chain variable region with a substitution, deletion or addition of 1 to 5 amino acids in the constituent amino acid sequence, which includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,
(x-f)
an antibody having the amino acid sequence of the light chain variable region with a substitution, deletion or addition of 1 to 3 amino acids in the constituent amino acid sequence, which includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and having the amino acid sequence of the heavy chain variable region with a substitution, deletion or addition of 1 to 3 amino acids in the constituent amino acid sequence, which includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3, and
(x-g)
an antibody having the amino acid sequence of the light chain variable region with a substitution, deletion or addition of 1 or 2 amino acids in the constituent amino acid sequence, which includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and having the amino acid sequence of the heavy chain variable region with a substitution, deletion or addition of 1 or 2 amino acids in the constituent amino acid sequence, which includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3.

The combination of amino acid sequences for each CDR of (x-a) to (x-g) above may be any combination, for example, such as those listed in Table 2. The same applies for (y-a) to (y-g) below as well.

[Table 2]

| (Table 2) Light and heavy chain CDR amino acid sequence combination example 2 | | | | | | |
|---|---|---|---|---|---|---|
| | Light chain | | | Heavy chain | | |
| | CDR1 | CDR2 | CDR3 | CDR1 | CDR2 | CDR3 |
| SEQ ID NO: | 8 | 10 | 12 | 13 | 15 | 17 |
| | 8 | 10 | 12 | 14 | 16 | 18 |
| | 9 | 11 | 12 | 13 | 15 | 17 |
| | 9 | 11 | 12 | 14 | 16 | 18 |

In the present invention, a preferred embodiment of the invention when the antibody is a Fab that is a humanized antibody and an anti-human transferrin receptor antibody, is the following:
(y) an antibody that is a Fab, in which the light chain includes the amino acid sequence set forth as SEQ ID NO: 22, and the heavy chain includes the amino acid sequence set forth as SEQ ID NO: 23. Here, the light chain includes the amino acid sequence set forth as SEQ ID NO: 20 as the variable region, and the heavy chain includes the amino acid sequence set forth as SEQ ID NO: 21 as the variable region. In the invention, the heavy chain comprising the Fab region is referred to as the "Fab heavy chain". The heavy chain composed of the amino acid sequence set forth as SEQ ID NO: 23 is therefore the Fab heavy chain.

Preferred embodiments where the antibody is a humanized antibody and an anti-human transferrin receptor antibody are not limited to (y). For example, an antibody wherein the amino acid sequence of the light chain has identity of not lower than 80% to the amino acid sequence of the light chain of (y) above, and wherein the amino acid sequence of the heavy chain has identity of not lower than 80% to the amino acid sequence of the heavy chain of (y) above, may also be used for the present invention so long as it has affinity for hTfR.

Further, an antibody in which the amino acid sequence of the light chain has identity of not lower than 85% to the amino acid sequence of the light chain of (y) above, and the amino acid sequence of the heavy chain has identity of not lower than 85% to the amino acid sequence of the heavy chain of (y) above,

an antibody in which the amino acid sequence of the light chain has identity of not lower than 90% to the amino acid sequence of the light chain of (y) above, and the amino acid sequence of the heavy chain has identity of not lower than 90% to the amino acid sequence of the heavy chain of (y) above, and

an antibody in which the amino acid sequence of the light chain has identity of not lower than 95% to the amino acid sequence of the light chain of (y) above, and the amino acid sequence of the heavy chain has identity of not lower than 95% to the amino acid sequence of the heavy chain of (y) above, can also be used in the present invention, so long as the antibody still has affinity with hTfR.

Antibodies wherein the amino acid sequence of the light chain has identity to the amino acid sequence of the light chain of (y) above and the amino acid sequence of the heavy chain has identity to the amino acid sequence of the heavy chain of (y) above, include the following:

(y-a) an antibody wherein the light chain includes an amino acid sequence having sequence identity of not less than 80% to the amino acid sequence set forth as SEQ ID NO: 22, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain includes an amino acid sequence having sequence identity of not less than 80% to the amino acid sequence set forth as SEQ ID NO: 23, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,

(y-b) an antibody wherein the light chain includes an amino acid sequence having sequence identity of not less than 85% to the amino acid sequence set forth as SEQ ID NO: 22, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain includes an amino acid sequence having sequence identity of not less than 85% to the amino acid sequence set forth as SEQ ID NO: 23, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,

(y-c) an antibody wherein the light chain includes an amino acid sequence having sequence identity of not less than 90% to the amino acid sequence set forth as SEQ ID NO: 22, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain includes an amino acid sequence having sequence identity of not less than 90% to the amino acid sequence set forth as SEQ ID NO: 23, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,

(y-d) an antibody wherein the light chain includes an amino acid sequence having sequence identity of not less than 95% to the amino acid sequence set forth as SEQ ID NO: 22, and includes the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and the heavy chain includes an amino acid sequence having sequence identity of not less than 95% to the amino acid sequence set forth as SEQ ID NO: 23, and includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3.

When the antibody is a Fab that is a humanized antibody and an anti-human transferrin receptor antibody, preferred embodiments of the antibody to be used for the invention include those having the following amino acid sequences:

the amino acid sequence of the light chain of (y) above with a substitution, deletion or addition of 1 to 5 amino acids in the constituent amino acid sequence, and the amino acid sequence of the heavy chain of (y) above with a substitution, deletion or addition of 1 to 5 amino acids in the constituent amino acid sequence,

the amino acid sequence of the light chain of (y) above with a substitution, deletion or addition of 1 to 3 amino acids in the constituent amino acid sequence, and the amino acid sequence of the heavy chain of (y) above with a substitution, deletion or addition of 1 to 3 amino acids in the constituent amino acid sequence, and

the amino acid sequence of the light chain of (y) above with a substitution, deletion or addition of 1 or 2 amino acids in the constituent amino acid sequence, and the amino acid sequence of the heavy chain of (y) above with a substitution, deletion or addition of 1 or 2 amino acids in the constituent amino acid sequence,

so long as the antibody still has affinity for hTfR.

Antibodies wherein the amino acid sequence of the light chain has the amino acid sequence of the light chain of (y) above with a substitution, deletion or addition of an amino acid in the constituent amino acid sequence and the amino acid sequence of the heavy chain of (y) above with a substitution, deletion or addition of an amino acid in the constituent amino acid sequence, include the following:

(y-e)

an antibody having the amino acid sequence of the light chain with a substitution, deletion or addition of 1 to 5 amino acids in the constituent amino acid sequence, and including the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and having the amino acid sequence of the heavy chain with a substitution, deletion or addition of 1 to 5 amino acids in the constituent amino acid sequence, which includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3,

(y-f)

an antibody having the amino acid sequence of the light chain with a substitution, deletion or addition of 1 to 3 amino acids in the constituent amino acid sequence, and including the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and having the amino acid sequence of the heavy chain with a substitution, deletion or addition of 1 to 3 amino acids in the constituent amino acid sequence, which includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3, and

(y-g)

an antibody having the amino acid sequence of the light chain with a substitution, deletion or addition of 1 or 2 amino acids in the constituent amino acid sequence, and including the amino acid sequence of SEQ ID NO: 8 or 9 as CDR1, the amino acid sequence of SEQ ID NO: 10 or 11 as CDR2 and the amino acid sequence of SEQ ID NO: 12 as CDR3, and having the amino acid sequence of the heavy chain with a substitution, deletion or addition of 1 or 2 amino acids in the constituent amino acid sequence, which includes the amino acid sequence of SEQ ID NO: 13 or 14 as CDR1, the amino acid sequence of SEQ ID NO: 15 or 16 as CDR2 and the amino acid sequence of SEQ ID NO: 17 or 18 as CDR3.

When the antibody is a humanized anti-human transferrin receptor antibody and the human lysosomal enzyme is human α-L-iduronidase (hIDUA), the preferable embodiments of the fusion proteins include following:
a fusion protein comprising the light chain of humanized anti-hTfR antibody having the amino acid sequence set forth as SEQ ID NO: 22, and the human α-L-iduronidase set forth as SEQ ID NO: 6 linked to the C-terminus of the Fab heavy chain of humanized anti-hTfR antibody having the amino acid sequence set forth as SEQ ID NO: 23, via the linker set forth as SEQ ID NO: 4.

When the antibody is a humanized anti-human transferrin receptor antibody and the human lysosomal enzyme is human α-L-iduronidase (hIDUA), the preferable embodiments of the fusion proteins include following:

a fusion protein comprising the light chain of humanized anti-hTfR antibody having the amino acid sequence set forth as SEQ ID NO: 22, and the Fab heavy chain of the humanized anti-hTfR antibody linked at the C-terminus with human α-L-iduronidase set forth as SEQ ID NO: 6 via the linker having the amino acid sequence set forth as SEQ ID NO: 4, such that the fusion protein as a whole has the amino acid sequence set forth as SEQ ID NO: 27.

In the present invention, the protein having physiological activity can be produced, for example, by culturing of mammalian cells artificially manipulated so as to produce the protein by expression or overexpression of a gene encoding the protein. The gene to be overexpressed in mammalian cells that produce the protein will generally be transferred into the mammalian cells by transformation with an expression vector incorporating the gene. The mammalian cells used are not particularly limited but are preferably human, mouse or Chinese hamster cells, and most preferably CHO cells, derived from Chinese hamster ovary cells. According to the invention, a "protein" is a recombinant protein secreted into a medium when mammalian cells that produce the protein are cultured.

According to one embodiment of the present invention, the aqueous pharmaceutical composition comprises a protein having physiological activity as the active ingredient, and two or more different nonionic surfactants. The aqueous pharmaceutical composition may further comprise one or more neutral salts, disaccharides or buffering agents.

The two or more different nonionic surfactants in the aqueous pharmaceutical composition are not particularly limited so long as they are pharmaceutically acceptable, and suitable nonionic surfactants include polysorbates and poloxamers. Examples of polysorbates include polysorbate 20 and polysorbate 80. Suitable poloxamers include polyoxyethylene(42) polyoxypropylene(67) glycol, polyoxyethylene(54) polyoxypropylene(39) glycol, polyoxyethylene(196) polyoxypropylene(67) glycol, polyoxyethylene(42) polyoxypropylene(67) glycol, polyoxyethylene(3) polyoxypropylene(17) glycol, polyoxyethylene(20) polyoxypropylene(20) glycol and polyoxyethylene(120) polyoxypropylene(40) glycol, with polyoxyethylene(160) polyoxypropylene(30) glycol being especially preferred. Polyoxyethylene(160) polyoxypropylene(30) glycol is synonymous with poloxamer 188.

When the aqueous pharmaceutical composition comprises two different nonionic surfactants, the preferred combination of nonionic surfactants is one is a polysorbate and the other is a poloxamer. For example, preferred combinations are polysorbate 20 and poloxamer 188, or polysorbate 80 and poloxamer 188, especially the combination of polysorbate 80 and poloxamer 188is preferrable. These combinations may be further combined with other type of polysorbate or poloxamer.

The respective concentrations of two of the two or more different nonionic surfactants in the aqueous pharmaceutical composition are preferably 0.105 to 0.6 mg/mL and 0.005 to 1.5 mg/mL, more preferably 0.1 to 0.5 mg/mL and 0.025 to 1.0 mg/mL, even more preferably 0.15 to 0.45 mg/mL and 0.05 to 1.0 mg/mL, and yet more preferably 0.25 to 0.45 mg/mL and 0.05 to 0.15 mg/mL for example, such as 0.325 mg/mL and 0.075 mg/mL. Examples for the respective concentrations include 0.162 mg/mL and 0.075 mg/mL, 0.130 mg/mL and 0.075 mg/mL, 0.108 mg/mL and 0.075 mg/mL, and 0.08 mg/mL and 0.075 mg/mL.

When the aqueous pharmaceutical composition comprises a polysorbate and a poloxamer as two different nonionic surfactants, the concentration of the polysorbate is preferably 0.005 to 1.5 mg/mL, more preferably 0.025 to 1.0 mg/mL, even more preferably 0.05 to 1.0 mg/mL and yet more preferably 0.05 to 0.15 mg/mL, for example, such as 0.075 mg/mL. The concentration of the poloxamer in this case is preferably 0.105 to 0.6 mg/mL, more preferably 0.1 to 0.5 mg/mL and even more preferably 0.15 to 0.45 mg/mL, for example, such as 0.325 mg/mL. Further examples include 0.162 mg/mL, 0.130 mg/mL, 0.108 mg/mL and 0.08 mg/mL.

When the aqueous pharmaceutical composition comprises polysorbate 80 and poloxamer 188 as two different nonionic surfactants, the concentration of the polysorbate 80 is preferably 0.005 to 0.15 mg/mL, more preferably 0.025 to 1.0 mg/mL and even more preferably 0.05 to 1.0 mg/mL, for example, such as 0.075 mg/mL. The concentration of the poloxamer 188 in this case is preferably 0.105 to 0.6 mg/mL, more preferably 0.1 to 0.5 mg/mL and even more preferably 0.15 to 0.45 mg/mL, for example, such as 0.325 mg/mL, with further examples including 0.162 mg/mL, 0.130 mg/mL, 0.108 mg/mL and 0.08 mg/mL. For example, the concentration of polysorbate 80 may be 0.05 to 1.0 mg/mL and the concentration of poloxamer 188 may be 0.15 to 0.45 mg/mL. A specific example is that the concentration of polysorbate 80 is 0.075 mg/mL and the concentration of poloxamer 188 is 0.325 mg/mL. Further examples include the concentration of polysorbate 80 concentration is 0.075 mg/mL and the concentration of poloxamer 188 is 0.162 mg/mL, the concentration of polysorbate 80 is 0.075 mg/mL and the concentration of poloxamer 188 is 0.130 mg/mL, the concentration of polysorbate 80 is 0.075 mg/mL and the concentration of poloxamer 188 is 0.108 mg/mL, and the concentration of polysorbate 80 is 0.075 mg/mL and the concentration of poloxamer 188 concentration is 0.08 mg/mL.

There are no particular limitations on the neutral salt in the aqueous pharmaceutical composition so long as it is pharmaceutically acceptable, and suitable neutral salts include sodium chloride and magnesium chloride, with sodium chloride being especially preferred.

The concentration of the neutral salt in the aqueous pharmaceutical composition is preferably 0.3 to 1.2 mg/mL, more preferably 0.5 to 1.0 mg/mL and even more preferably 0.7 to 0.9 mg/mL, for example 0.8 mg/mL.

There are no particular limitations on the disaccharide in the aqueous pharmaceutical composition so long as it is pharmaceutically acceptable, and suitable disaccharides include trehalose, sucrose, maltose, lactose and their combinations, with sucrose being especially preferred.

The concentration of the disaccharide in the aqueous pharmaceutical composition is preferably 50 to 100 mg/mL, more preferably 55 to 95 mg/mL and even more preferably 60 to 90 mg/mL, for example, 75 mg/mL.

There are no particular restrictions on the buffering agent in the aqueous pharmaceutical composition so long as it is pharmaceutically acceptable, but it is preferably citrate buffer, phosphate buffer, glycine buffer, histidine buffer, carbonate buffer, acetate buffer, or combinations of the foregoing. The concentration of the buffering agent in the aqueous pharmaceutical composition is preferably 3 to 30 mM, more preferably 10 to 30 mM and even more preferably 15 to 25 mM, for example, 20 mM. The pH of the aqueous pharmaceutical composition as adjusted by the buffering agent is preferably 4.5 to 7.0, more preferably 4.5 to 6.5, even more preferably 5.0 to 6.0 and yet more preferably 5.2 to 5.8, for example, 5.5. When citrate buffer is used as the buffering agent, the concentration of the citrate buffer in the aqueous pharmaceutical composition is preferably 3 to 30 mM, more preferably 10 to 30 mM and even more preferably 15 to 25 mM, for example, 20 mM. The pH of the aqueous pharmaceutical composition as adjusted by the citrate buffer is preferably 4.5 to 7.0, more preferably 4.5 to 6.5, even more preferably 5.0 to 6.0 and yet more preferably 5.2 to 5.8, for example, 5.5.

Preferred compositions for the aqueous pharmaceutical composition of the present invention include:

(A) a composition wherein the concentration of the protein having physiological activity is 0.5 to 20 mg/mL, the concentration of the neutral salt is 0.3 to 1.2 mg/mL, the concentration of the disaccharide is 50 to 100 mg/mL, the respective concentrations of two different nonionic surfactants among the two or more different nonionic surfactants are 0.105 to 0.6 mg/mL and 0.005 to 1.5 mg/mL, the concentration of the buffering agent is 3 to 30 mM, and the pH is 4.5 to 6.5,

(B) a composition wherein the concentration of the protein having physiological activity is 1.0 to 10 mg/mL, the concentration of the neutral salt is 0.5 to 1.0 mg/mL, the concentration of the disaccharide is 55 to 95 mg/mL, the respective concentrations of two different nonionic surfactants among the two or more different nonionic surfactants are 0.1 to 0.5 mg/mL and 0.025 to 1.0 mg/mL, the concentration of the buffering agent is 10 to 30 mM, and the pH is 5.0 to 6.0, and

(C) the concentration of the protein having physiological activity is 2.0 to 10 mg/mL, the concentration of the neutral salt is 0.7 to 0.9 mg/mL, the concentration of the disaccharide is 60 to 90 mg/mL, the respective concentrations of two different nonionic surfactants among the two or more different nonionic surfactants are 0.15 to 0.45 mg/mL and 0.05 to 0.15 mg/mL, the concentration of the buffering agent is 15 to 25 mM, and the pH is 5.2 to 5.8.

In the aqueous pharmaceutical compositions of (A) to (C) above, the protein having physiological activity is a fusion protein of humanized anti-hTfR antibody and hIDUA, for example. The following is a preferred form of the fusion protein of humanized anti-hTfR antibody and hIDUA:
a fusion protein comprising the light chain of humanized anti-hTfR antibody having the amino acid sequence set forth as SEQ ID NO: 22, and the human α-L-iduronidase set forth as SEQ ID NO: 6 linked to the C-terminus of the Fab heavy chain of humanized anti-hTfR antibody having the amino acid sequence set forth as SEQ ID NO: 23, via the linker set forth as SEQ ID NO: 4.

When the protein having physiological activity in the aqueous pharmaceutical compositions of (A) to (C) above is a fusion protein of humanized anti-hTfR antibody and hIDUA, the following is a more preferred form of the fusion protein: a fusion protein wherein the light chain of humanized anti-hTfR antibody has the amino acid sequence set forth as SEQ ID NO: 22, and the Fab heavy chain of the humanized anti-hTfR antibody is linked at the C-terminus with human α-L-iduronidase set forth as SEQ ID NO: 6 via the linker having the amino acid sequence set forth as SEQ ID NO: 4, such that the fusion protein as a whole has the amino acid sequence set forth as SEQ ID NO: 27.

The preferred concentration for a fusion protein of humanized anti-hTfR antibody and hIDUA in the aqueous pharmaceutical compositions of (A) to (C) above is 0.5 to 20 mg/mL, 1.0 to 10 mg/mL, 2.0 to 10 mg/mL or 2.0 to 6.0 mg/mL, suitably adjusted to 2.5 mg/mL or 5.0 mg/mL.

The neutral salt in the aqueous pharmaceutical compositions of (A) to (C) above is not particularly limited so long as it is pharmaceutically acceptable, but sodium chloride is preferred. When sodium chloride is used as the neutral salt, its concentration is preferably 0.3 to 1.2 mg/mL, more preferably 0.5 to 1.0 mg/mL and even more preferably 0.7 to 0.9 mg/mL, for example, 0.8 mg/mL.

The disaccharide in the aqueous pharmaceutical compositions of (A) to (C) above is not particularly limited so long as it is pharmaceutically acceptable, but sucrose is preferred. When sucrose is used as the disaccharide, its concentration is preferably 50 to 100 mg/mL, more preferably 55 to 95 mg/mL and even more preferably 60 to 90 mg/mL, for example, 75 mg/mL.

The two or more different nonionic surfactants in the aqueous pharmaceutical compositions of (A) to (C) above are not particularly limited so long as they are pharmaceutically acceptable, and suitable nonionic surfactants include polysorbates and poloxamers. Examples of polysorbates include polysorbate 20 and polysorbate 80. Suitable poloxamers include polyoxyethylene(42) polyoxypropylene(67) glycol, polyoxyethylene(54) polyoxypropylene(39) glycol, polyoxyethylene(196) polyoxypropylene(67) glycol, polyoxyethylene(42) polyoxypropylene(67) glycol, polyoxyethylene(3) polyoxypropylene(17) glycol, polyoxyethylene(20) polyoxypropylene(20) glycol and polyoxyethylene(120) polyoxypropylene(40) glycol, and among them polyoxyethylene(160) polyoxypropylene(30) glycol is especially preferrable. When the aqueous pharmaceutical composition comprises two different nonionic surfactants, the preferred combination of nonionic surfactants is a polysorbate and a poloxamer. For example, preferred combinations are polysorbate 20 and poloxamer 188, or polysorbate 80 and poloxamer 188. The combination of polysorbate 80 and poloxamer 188 is especially preferrable. These combinations may be further combined with other type of polysorbates or poloxamers. The respective concentrations of the two or more different nonionic surfactants in the aqueous pharmaceutical composition are preferably 0.105 to 0.6 mg/mL and 0.005 to 1.5 mg/mL, more preferably 0.1 to 0.5 mg/mL and 0.025 to 1.0 mg/mL, even more preferably 0.15 to 0.45 mg/mL and 0.05 to 1.0 mg/mL, and yet more preferably 0.25 to 0.45 mg/mL and 0.05 to 0.15 mg/mL, for example, 0.325 mg/mL and 0.075 mg/mL. Examples for the respective concentrations include 0.162 mg/mL and 0.075 mg/mL, 0.130 mg/mL and 0.075 mg/mL, 0.108 mg/mL and 0.075 mg/mL, and 0.08 mg/mL and 0.075 mg/mL. When the aqueous pharmaceutical composition comprises a polysorbate and a poloxamer as two different nonionic surfactants, the concentration of the polysorbate is preferably 0.005 to 1.5 mg/mL, more preferably 0.025 to 1.0 mg/mL, even more preferably 0.05 to 1.0 mg/mL and yet more preferably 0.05 to 0.15 mg/mL, for example, 0.075 mg/mL. The concentration of the poloxamer in this case is preferably 0.105 to 0.6 mg/mL, more preferably 0.1 to 0.5 mg/mL and even more preferably 0.15 to 0.45 mg/mL, for example, 0.325 mg/mL. Further examples include 0.162 mg/mL, 0.130 mg/mL, 0.108 mg/mL and 0.08 mg/mL.

The buffering agent used in the aqueous pharmaceutical compositions of (A) to (C) above is not particularly limited so long as it is pharmaceutically acceptable, but citrate buffer is preferred. When citrate buffer is used as the buffering agent, its concentration is preferably 3 to 30 mM, more preferably 10 to 30 mM and even more preferably 15 to 25 mM, for example, 20 mM. The pH of the aqueous pharmaceutical composition as adjusted by the buffering agent is preferably 4.5 to 6.5, more preferably 5.0 to 6.0, even more preferably 5.2 to 5.8, for example, 5.5.

When the protein having physiological activity is a fusion protein of an antibody and a human lysosomal enzyme, an example of a suitable composition for the aqueous pharmaceutical composition is a composition in which the fusion protein is a fusion protein of humanized anti-hTfR antibody and hIDUA at a concentration of 1 to 10 mg/mL, the concentration of the neutral salt (especially sodium chloride) is 0.3 to 1.2 mg/mL, the concentration of the disaccharide (especially sucrose) is 50 to 100 mg/mL, the concentration of the polysorbate (especially polysorbate 80) is 0.025 to 1.0 mg/mL, the concentration of the poloxamer (especially poloxamer 188) is 0.1 to 0.5 mg/mL, and the concentration of the citrate buffer is 10 to 30 mM. As a more specific example, the concentration of the fusion protein of humanized anti-hTfR antibody and hIDUA is 1 to 10 mg/mL, the concentration of sodium chloride is 0.5 to 1.0 mg/mL, the concentration of sucrose is 55 to 95 mg/mL, the concentration of the polysorbate (especially polysorbate 80) is 0.05 to 1.0 mg/mL, the concentration of the poloxamer (especially poloxamer 188) is 0.15 to 0.45 mg/mL, and the concentration of the citrate buffer is 15 to 25 mM. As another specific example, the concentration of the fusion protein of humanized anti-hTfR antibody and hIDUA is 1 to 10 mg/mL, the concentration of sodium chloride is 0.7 to 0.9 mg/mL, the concentration of sucrose is 60 to 90 mg/mL, the concentration of the polysorbate (especially polysorbate 80) is 0.05 to 0.15 mg/mL, the concentration of the poloxamer (especially poloxamer 188) is 0.15 to 0.45 mg/mL, and the concentration of the citrate buffer is 15 to 25 mM. As yet another specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.325 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.162 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.130 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.108 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.08 mg/mL, and the concentration of the citrate buffer is 20 mM.

The aqueous pharmaceutical composition of the present invention in which the active ingredient is a protein having physiological activity can be stably stored in a dark environment at a temperature of 2 to 8°C, for a period of preferably 3 months, more preferably 1 year, even more preferably 2 years and even more preferably 3 years or longer, such as 4 years, 5 years or 6 years. The aqueous pharmaceutical composition of the invention can be stably stored for preferably 3 months and more preferably 6 months in a dark environment at a temperature of 22 to 28°C.

The aqueous pharmaceutical composition of the present invention in which the active ingredient is a protein having physiological activity can be administered by intravenous, intramuscular, intraperitoneal or subcutaneous injection. The aqueous pharmaceutical composition of the invention may be filled into a vial, or provided as a prefilled formulation in a syringe. There are no particular limitations on the material of the container such as a syringe or vial for filling of the aqueous pharmaceutical composition, but borosilicate glass is suitable, as well as a hydrophobic resin such as a cycloolefin copolymer which is a copolymer of a cyclic olefin and an olefin, a cycloolefin ring-opening polymer, or a hydrogenated cycloolefin ring-opening polymer.

The lyophilized pharmaceutical composition according to one embodiment of the present invention comprises a protein having physiological activity, as the active ingredient, and two or more different nonionic surfactants. The lyophilized pharmaceutical composition may further comprise one or more neutral salts, disaccharides or buffering agents.

The two or more different nonionic surfactants in the lyophilized pharmaceutical composition are not particularly limited so long as they are pharmaceutically acceptable, and suitable nonionic surfactants include polysorbates and poloxamers. Examples of polysorbates include polysorbate 20 and polysorbate 80. Suitable poloxamers include polyoxyethylene(42) polyoxypropylene(67) glycol, polyoxyethylene(54) polyoxypropylene(39) glycol, polyoxyethylene(196) polyoxypropylene(67) glycol, polyoxyethylene(42) polyoxypropylene(67) glycol, polyoxyethylene(3) polyoxypropylene(17) glycol, polyoxyethylene(20) polyoxypropylene(20) glycol and polyoxyethylene(120) polyoxypropylene(40) glycol. Among them polyoxyethylene(160) polyoxypropylene(30) glycol is especially preferrable. Polyoxyethylene(160) polyoxypropylene(30) glycol is synonymous with poloxamer 188.

When the lyophilized pharmaceutical composition comprises two different nonionic surfactants, the preferred combination of nonionic surfactants is a polysorbate and a poloxamer. For example, preferred combinations are polysorbate 20 and poloxamer 188 or polysorbate 80 and poloxamer 188, and the combination of polysorbate 80 and poloxamer 188 is especially preferrable. These combinations may be further combined with other type of polysorbates or poloxamers.

According to one embodiment of the invention, the lyophilized pharmaceutical composition is used after dissolution in an aqueous solvent. Purified water and physiological saline are suitable for use as aqueous solvents, but there is no limitation to these. One problem encountered when dissolving a lyophilized pharmaceutical composition in an aqueous solvent is that a portion of the lyophilized component may fail to dissolve, but this problem does not occur with the lyophilized pharmaceutical composition of the present invention. A solution obtained by dissolving the lyophilized pharmaceutical composition with an aqueous solvent is also considered to be an aqueous pharmaceutical composition of the invention. Such an aqueous pharmaceutical composition can be stably stored for preferably 1 day and more preferably 1 week in a dark environment at a temperature of 2 to 8°C. The lyophilized pharmaceutical composition can be stably stored as a lyophilized pharmaceutical composition in a dark environment at a temperature of 2 to 8°C, for a period of preferably 3 months, more preferably 1 year, even more preferably 2 years and even more preferably 3 years or longer, such as 4 years, 5 years or 6 years. The lyophilized pharmaceutical composition can also be stably stored for preferably 3 months and more preferably 6 months in a dark environment at a temperature of 22 to 28°C.

The respective concentrations of two of the two or more different nonionic surfactants in the aqueous pharmaceutical composition obtained by dissolving the lyophilized pharmaceutical composition are preferably 0.105 to 0.6 mg/mL and 0.005 to 1.5 mg/mL, more preferably 0.1 to 0.5 mg/mL and 0.025 to 1.0 mg/mL, even more preferably 0.15 to 0.45 mg/mL and 0.05 to 1.0 mg/mL, and yet more preferably 0.15 to 0.45 mg/mL and 0.05 to 0.15 mg/mL, for example, 0.325 mg/mL and 0.075 mg/mL. Examples for the respective concentrations include 0.162 mg/mL and 0.075 mg/mL, 0.130 mg/mL and 0.075 mg/mL, 0.108 mg/mL and 0.075 mg/mL, and 0.08 mg/mL and 0.075 mg/mL.

When the lyophilized pharmaceutical composition comprises a polysorbate and a poloxamer as two different nonionic surfactants, the concentration of the polysorbate in the aqueous pharmaceutical composition obtained by its dissolution is preferably 0.005 to 1.5 mg/mL, more preferably 0.025 to 1.0 mg/mL, even more preferably 0.05 to 1.0 mg/mL and yet more preferably 0.05 to 0.15 mg/mL, such as 0.075 mg/mL, for example. The concentration of the poloxamer in this case is preferably 0.105 to 0.6 mg/mL, more preferably 0.1 to 0.5 mg/mL and even more preferably 0.15 to 0.45 mg/mL, for example, 0.325 mg/mL. Further examples include 0.162 mg/mL, 0.130 mg/mL, 0.108 mg/mL and 0.08 mg/mL.

When the lyophilized pharmaceutical composition comprises polysorbate 80 and poloxamer 188 as two different nonionic surfactants, the concentration of the polysorbate 80 in the aqueous pharmaceutical composition obtained by dissolving it is preferably 0.005 to 0.15 mg/mL, more preferably 0.025 to 1.0 mg/mL and even more preferably 0.05 to 1.0 mg/mL, for example, 0.075 mg/mL. The concentration of poloxamer 188 in this case is preferably 0.105 to 0.6 mg/mL, more preferably 0.1 to 0.5 mg/mL and even more preferably 0.15 to 0.45 mg/mL, such as 0.325 mg/mL, for example. Further examples include 0.162 mg/mL, 0.130 mg/mL, 0.108 mg/mL and 0.08 mg/mL. For example, the concentration of polysorbate 80 may be 0.05 to 1.0 mg/mL and the concentration of poloxamer 188 may be 0.15 to 0.45 mg/mL. A specific example is that the concentration of polysorbate 80 is 0.075 mg/mL and the concentration of poloxamer 188 is 0.325 mg/mL. Further examples include a polysorbate 80 concentration of 0.075 mg/mL and a poloxamer 188 concentration of 0.162 mg/mL, a polysorbate 80 concentration of 0.075 mg/mL and a poloxamer 188 concentration of 0.130 mg/mL, a polysorbate 80 concentration of 0.075 mg/mL and a poloxamer 188 concentration of 0.108 mg/mL, and a polysorbate 80 concentration of 0.075 mg/mL and a poloxamer 188 concentration of 0.08 mg/mL.

There are no particular limitations on the neutral salt in the lyophilized pharmaceutical composition so long as it is pharmaceutically acceptable, and suitable neutral salts include sodium chloride and magnesium chloride, with sodium chloride being especially preferred.

When the lyophilized pharmaceutical composition comprises a neutral salt, the concentration of the neutral salt in the aqueous pharmaceutical composition obtained by dissolving it is preferably 0.3 to 1.2 mg/mL, more preferably 0.5 to 1.0 mg/mL and even more preferably 0.7 to 0.9 mg/mL, for example, 0.8 mg/mL.

There are no particular limitations on the disaccharide in the lyophilized pharmaceutical composition so long as it is pharmaceutically acceptable, and suitable disaccharides include trehalose, sucrose, maltose, lactose and their combinations, with sucrose being especially preferred.

When the lyophilized pharmaceutical composition comprises a disaccharide, the concentration of the disaccharide in the aqueous pharmaceutical composition obtained by dissolving it is preferably 50 to 100 mg/mL, more preferably 55 to 95 mg/mL and even more preferably 60 to 90 mg/mL, for example, 75 mg/mL.

There are no particular limitations on the buffering agent in the lyophilized pharmaceutical composition so long as it is pharmaceutically acceptable, but it is preferably citrate buffer, phosphate buffer, glycine buffer, histidine buffer, carbonate buffer, acetate buffer, or combinations of the foregoing. When the lyophilized pharmaceutical composition comprises a buffering agent, the concentration of the buffering agent in the aqueous pharmaceutical composition obtained by dissolving it is preferably 3 to 30 mM, more preferably 10 to 30 mM and even more preferably 15 to 25 mM, for example, such as 20 mM. The pH of the aqueous pharmaceutical composition as adjusted by the buffering agent is preferably 4.5 to 7.0, more preferably 4.5 to 6.5, even more preferably 5.0 to 6.0 and yet more preferably 5.2 to 5.8, for example, 5.5. When citrate buffer is used as the buffering agent, the concentration of the citrate buffer in the aqueous pharmaceutical composition is preferably 3 to 30 mM, more preferably 10 to 30 mM and even more preferably 15 to 25 mM, for example, 20 mM. The pH of the aqueous pharmaceutical composition as adjusted by the citrate buffer is preferably 4.5 to 7.0, more preferably 4.5 to 6.5, even more preferably 5.0 to 6.0 and yet more preferably 5.2 to 5.8, for example, 5.5.

Preferred compositions for the lyophilized pharmaceutical composition of the present invention, as aqueous pharmaceutical compositions obtained by dissolving them, include:

(D) a composition wherein the concentration of the protein having physiological activity is 0.5 to 20 mg/mL, the concentration of the neutral salt is 0.3 to 1.2 mg/mL, the concentration of the disaccharide is 50 to 100 mg/mL, the respective concentrations of two different nonionic surfactants from among the two or more different nonionic surfactants are 0.105 to 0.6 mg/mL and 0.005 to 1.5 mg/mL, the concentration of the buffering agent is 3 to 30 mM, and the pH is 4.5 to 6.5,

(E) a composition wherein the concentration of the protein having physiological activity is 1.0 to 10 mg/mL, the concentration of the neutral salt is 0.5 to 1.0 mg/mL, the concentration of the disaccharide is 55 to 95 mg/mL, the respective concentrations of two different nonionic surfactants from among the two or more different nonionic surfactants are 0.1 to 0.5 mg/mL and 0.025 to 1.0 mg/mL, the concentration of the buffering agent is 10 to 30 mM, and the pH is 5.0 to 6.0, and

(F) the concentration of the protein having physiological activity is 2.0 to 10 mg/mL, the concentration of the neutral salt is 0.7 to 0.9 mg/mL, the concentration of the disaccharide is 60 to 90 mg/mL, the respective concentrations of two different nonionic surfactants from among the two or more different nonionic surfactants are 0.15 to 0.45 mg/mL and 0.05 to 0.15 mg/mL, the concentration of the buffering agent is 15 to 25 mM, and the pH is 5.2 to 5.8.

In (D) to (F) above, the protein having physiological activity is, for example, a fusion protein of humanized anti-hTfR antibody and hIDUA. The following is a preferred form of the fusion protein of humanized anti-hTfR antibody and hIDUA:

a fusion protein comprising the light chain of humanized anti-hTfR antibody having the amino acid sequence set forth as SEQ ID NO: 22, and the human α-L-iduronidase set forth as SEQ ID NO: 6 linked to the C-terminus of the Fab heavy chain of humanized anti-hTfR antibody having the amino acid sequence set forth as SEQ ID NO: 23, via the linker set forth as SEQ ID NO: 4.

When the protein having physiological activity of (D) to (F) above is a fusion protein of humanized anti-hTfR antibody and hIDUA, the following is a more preferred form of the fusion protein:

a fusion protein wherein the light chain of humanized anti-hTfR antibody has the amino acid sequence set forth as SEQ ID NO: 22, and the Fab heavy chain of the humanized anti-hTfR antibody is linked at the C-terminus with human α-L-iduronidase set forth as SEQ ID NO: 6 via the linker having the amino acid sequence set forth as SEQ ID NO: 4, such that the fusion protein as a whole has the amino acid sequence set forth as SEQ ID NO: 27.

The preferred concentration for a fusion protein of humanized anti-hTfR antibody and hIDUA in the aqueous pharmaceutical compositions of (D) to (F) above is 0.5 to 20 mg/mL, 1.0 to 10 mg/mL, 2.0 to 10 mg/mL or 2.0 to 6.0 mg/mL, and suitably is adjusted to 2.5 mg/mL or 5.0 mg/mL.

The neutral salt of (D) to (F) above is not particularly limited so long as it is pharmaceutically acceptable, but sodium chloride is preferred. When sodium chloride is used as the neutral salt, its concentration is preferably 0.3 to 1.2 mg/mL, more preferably 0.5 to 1.0 mg/mL and even more preferably 0.7 to 0.9 mg/mL, for example, 0.8 mg/mL.

The disaccharide of (D) to (F) above is not particularly limited so long as it is pharmaceutically acceptable, but sucrose is preferred. When sucrose is used as the disaccharide, its concentration is preferably 50 to 100 mg/mL, more preferably 55 to 95 mg/mL and even more preferably 60 to 90 mg/mL, for example, 75 mg/mL.

The two or more different nonionic surfactants of (D) to (F) above are not particularly limited so long as they are pharmaceutically acceptable, and suitable nonionic surfactants include polysorbates and poloxamers. Examples of polysorbates include polysorbate 20 and polysorbate 80. Suitable poloxamers include polyoxyethylene(42) polyoxypropylene(67) glycol, polyoxyethylene(54) polyoxypropylene(39) glycol, polyoxyethylene(196) polyoxypropylene(67) glycol, polyoxyethylene(42) polyoxypropylene(67) glycol, polyoxyethylene(3) polyoxypropylene(17) glycol, polyoxyethylene(20) polyoxypropylene(20) glycol and polyoxyethylene(120) polyoxypropylene(40) glycol, with polyoxyethylene(160) polyoxypropylene(30) glycol being especially preferred. When the composition of (D) to (F) above comprises two different nonionic surfactants, the preferred combination of nonionic surfactants is a polysorbate and a poloxamer. For example, preferred combinations are polysorbate 20 and poloxamer 188, or polysorbate 80 and poloxamer 188, with the combination of polysorbate 80 and poloxamer 188 being especially preferred. These combinations may be further combined with other type of polysorbates or poloxamers. The respective concentrations of two of the two or more different nonionic surfactants of (D) to (F) above are preferably 0.105 to 0.6 mg/mL and 0.005 to 1.5 mg/mL, more preferably 0.1 to 0.5 mg/mL and 0.025 to 1.0 mg/mL, even more preferably 0.15 to 0.45 mg/mL and 0.05 to 1.0 mg/mL, and yet more preferably 0.15 to 0.45 mg/mL and 0.05 to 0.15 mg/mL, for example, 0.325 mg/mL and 0.075 mg/mL. Examples for the respective concentrations include 0.162 mg/mL and 0.075 mg/mL, 0.130 mg/mL and 0.075 mg/mL, 0.108 mg/mL and 0.075 mg/mL, and 0.08 mg/mL and 0.075 mg/mL. When the composition of (D) to (F) above comprises a polysorbate and a poloxamer as two different nonionic surfactants, the concentration of the polysorbate is preferably 0.005 to 1.5 mg/mL, more preferably 0.025 to 1.0 mg/mL, even more preferably 0.05 to 1.0 mg/mL and yet more preferably 0.05 to 0.15 mg/mL, for example, 0.075 mg/mL. The concentration of the poloxamer in this case is preferably 0.105 to 0.6 mg/mL, more preferably 0.1 to 0.5 mg/mL and even more preferably 0.15 to 0.45 mg/mL, for example, 0.325 mg/mL. Further examples include 0.162 mg/mL, 0.130 mg/mL, 0.108 mg/mL and 0.08 mg/mL.

The buffering agent used in (D) to (F) above is not particularly limited so long as it is pharmaceutically acceptable, but citrate buffer is preferred. When citrate buffer is used as the buffering agent, its concentration is preferably 3 to 30 mM, more preferably 10 to 30 mM and even more preferably 15 to 25 mM, for example, 20 mM. The pH of the aqueous pharmaceutical composition as adjusted by the buffering agent is preferably 4.5 to 6.5, more preferably 5.0 to 6.0, even more preferably 5.2 to 5.8, for example, 5.5.

When the protein having physiological activity is a fusion protein of an antibody and a human lysosomal enzyme, an example of a suitable composition for the lyophilized pharmaceutical composition is a composition in which the fusion protein is a fusion protein of humanized anti-hTfR antibody and hIDUA, and in the aqueous pharmaceutical composition obtained by dissolving the lyophilized pharmaceutical composition, the concentration of the fusion protein is 1 to 10 mg/mL, the concentration of the neutral salt (especially sodium chloride) is 0.3 to 1.2 mg/mL, the concentration of the disaccharide (especially sucrose) is 50 to 100 mg/mL, the concentration of the polysorbate (especially polysorbate 80) is 0.025 to 1.0 mg/mL, the concentration of the poloxamer (especially poloxamer 188) is 0.1 to 0.5 mg/mL, and the concentration of the citrate buffer is 10 to 30 mM. As a more specific example, the concentration of the fusion protein of humanized anti-hTfR antibody and hIDUA is 1 to 10 mg/mL, the concentration of sodium chloride is 0.5 to 1.0 mg/mL, the concentration of sucrose is 55 to 95 mg/mL, the concentration of the polysorbate (especially polysorbate 80) is 0.05 to 1.0 mg/mL, the concentration of the poloxamer (especially poloxamer 188) is 0.15 to 0.45 mg/mL, and the concentration of the citrate buffer is 15 to 25 mM. As another specific example, the concentration of the fusion protein of humanized anti-hTfR antibody and hIDUA is 1 to 10 mg/mL, the concentration of sodium chloride is 0.7 to 0.9 mg/mL, the concentration of sucrose is 60 to 90 mg/mL, the concentration of the polysorbate (especially polysorbate 80) is 0.05 to 0.15 mg/mL, the concentration of the poloxamer (especially poloxamer 188) is 0.15 to 0.45 mg/mL, and the concentration of the citrate buffer is 15 to 25 mM. As yet another specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.325 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.162 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.130 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.108 mg/mL, and the concentration of the citrate buffer is 20 mM. As an even more specific example, the concentration of the fusion protein of humanized anti-hTfR antibody and hIDUA is 5 mg/mL, the concentration of sodium chloride is 0.8 mg/mL, the concentration of sucrose is 75 mg/mL, the concentration of polysorbate 80 is 0.075 mg/mL, the concentration of poloxamer 188 is 0.08 mg/mL, and the concentration of the citrate buffer is 20 mM.

The lyophilized preparation of the present invention can be provided as a kit packaged with a special solution for dissolving it. The lyophilized preparation may be administered to a patient intravenously, intramuscularly, intraperitoneally or subcutaneously after being dissolved in a special solution or purified water prior to use, for example. There are no particular limitations on the material of the container such as a syringe or vial for encapsulation or filling of the lyophilized preparation, but borosilicate glass is suitable, as well as a hydrophobic resin such as a cycloolefin copolymer which is a copolymer of a cyclic olefin and an olefin, a cycloolefin ring-opening polymer, or a hydrogenated cycloolefin ring-opening polymer.

The aqueous pharmaceutical composition according to one embodiment of the present invention has a polymer content ratio of preferably 0.5% or lower, more preferably 0.4% or lower and even more preferably 0.3% or lower after storage for 36 months in a dark environment at a temperature of 2 to 8°C. The aqueous pharmaceutical composition according to one embodiment of the invention also has a polymer content ratio and decomposition product content ratio of preferably 0.5% or lower and 1% or lower, more preferably 0.4% or lower and 0.8% or lower, and even more preferably 0.3% or lower and 0.6% or lower, respectively, after storage for 36 months in a dark environment at a temperature of 2 to 8°C.

The aqueous pharmaceutical composition according to one embodiment of the present invention can be stored after production in a dark environment at a temperature of 2 to 8°C, for preferably 24 months, more preferably 36 months, even more preferably 48 months and yet more preferably 72 months. In other words, it can be stored for long periods at a destination medical institution, in a state allowing it to be administered to a patient as an aqueous pharmaceutical composition.

The lyophilized pharmaceutical composition according to one embodiment of the invention has a polymer content ratio of preferably 0.5% or lower, more preferably 0.4% or lower and even more preferably 0.3% or lower after storage for 36 months in a dark environment at a temperature of 2 to 8°C. The aqueous pharmaceutical composition according to one embodiment of the invention also has a polymer content ratio and decomposition product content ratio of preferably 0.5% or lower and 0.1% or lower, more preferably 0.4% or lower and 0.07% or lower, and even more preferably 0.3% or lower and 0.05% or lower, respectively, after storage for 36 months in a dark environment at a temperature of 2 to 8°C.

The lyophilized pharmaceutical composition according to one embodiment of the invention can be stored after production in a dark environment at a temperature of 2 to 8°C, for preferably 24 months, more preferably 36 months, even more preferably 48 months and yet more preferably 72 months. In other words, it can be stored for long periods at a destination medical institution, in a state allowing it to be administered to a patient as a lyophilized pharmaceutical composition.

## Examples

The present invention will now be explained in greater detail through the following Examples, with the understanding that the invention is in no way limited by the Examples.

[Example 1] Construction of humanized anti-hTfR antibody-hIDUA fusion protein expression vector

A humanized anti-hTfR antibody-hIDUA fusion protein expression vector was constructed using genes encoding a light chain having the amino acid sequence set forth as SEQ ID NO: 22, and a heavy chain Fab region having the amino acid sequence set forth as SEQ ID NO: 23.

[Construction of pE-neo vector and pE-hygr vector]

The vector pEF/myc/nuc (Invitrogen Corp.) was digested with KpnI and NcoI, and the region containing EF-1 promoter and its first intron was cut out and blunt ended with T4 DNA polymerase. Separately, pCI-neo (Invitrogen Corp.) was digested with BglII and EcoRI, and after cutting off the region containing the CMV enhancer/promoter and intron, it was blunt ended with T4 DNA polymerase. To this the region containing the EF-1$\alpha$ promoter and its first intron (after blunt ending) was inserted to construct vector pE-neo. Vector pE-neo was digested with SfiI and BstXI, and an approximately 1 kbp region containing the neomycin resistance gene was cut off. With pcDNA3-1/Hygro(+) (Invitrogen Corp.) as template, primer Hyg-Sfi5' (SEQ ID NO: 13) and primer Hyg-BstX3' (SEQ ID NO: 14) were used for amplification of the hygromycin gene by PCR reaction. The amplified hygromycin gene was digested with SfiI and BstXI and inserted into vector pE-neo, to construct vector pE-hygr. Construction of vector pE-neo and vector pE-hygr was carried out with reference to patent literature (Japanese Patent No. 6279466).

[Construction of pE-IRES-GS-puro]

The expression vector pPGKIH (Miyahara M. et. al., J. Biol. Chem. 275, 613-618(2000)) was digested with restriction enzymes (XhoI and BamHI), and a DNA fragment containing the internal ribosome entry site (IRES) derived from murine encephalomyocarditis virus (EMCV), the hygromycin resistance gene (Hyg$^r$ gene), and the polyadenylated region (mPGKpA) of mouse phosphoglycerate kinase (mPGK) was cut out. The DNA fragment was inserted between the XhoI and BamHI sites of pBluescript SK(-) (Stratagene) to create pBSK(IRES-Hygr-mPGKpA).

With pBSK(IRES-Hygr-mPGKpA) as template, primer IRES5' (SEQ ID NO: 29) and primer IRES3' (SEQ ID NO: 30) were used for PCR amplification of a DNA fragment containing a portion of the IRES of EMCV. The DNA fragment was digested with restriction enzymes (XhoI and HindIII) and inserted between the XhoI and HindIII sites of pBSK(IRES-Hygr-mPGKpA), to create pBSK(NotI-IRES-Hygr-mPGKpA). After digesting pBSK(NotI-IRES-Hygr-mPGKpA) with restriction enzymes (NotI and BamHI), it was inserted between the NotI and BamHI sites of vector pE-hygr, to create plasmid pE-IRES-Hygr.

The expression vector pPGKIH was digested with EcoRI, and a DNA fragment comprising a nucleotide sequence including the mPGK promoter region (mPGKp) was cut out. The DNA fragment was inserted at the EcoRI site of pBluescript SK(-) (Stratagene) to create mPGK promoter/pBS(-). With mPGK promoter/pBS(-) as template, primer mPGKP5' (SEQ ID NO: 31) and primer mPGKP3' (SEQ ID NO: 32) were used for PCR amplification of a DNA fragment containing the mPGK promoter region (mPGKp). The DNA fragment was digested with restriction enzymes (BglII and EcoRI) and inserted between the BglII and EcoRI sites of pCI-neo(Promega) to create pPGK-neo. After digesting pE-IRES-Hygr with restriction enzymes (NotI and BamHI) to cut out a DNA fragment (IRES-Hygr), it was inserted between the NotI and BamHI sites of pPGK-neo, to create pPGK-IRES-Hygr.

cDNA was prepared from CHO-K1 cells, and a DNA fragment containing the GS gene was amplified by PCR using the cDNA as template, and using primer GS5' (SEQ ID NO: 33) and primer GS3' (SEQ ID NO: 34). After digesting the DNA fragment with restriction enzymes (BalI and BamHI), it was inserted between the BalI and BamHI sites of pPGK-IRES-Hygr, to create pPGK-IRES-GS-$\Delta$polyA.

With pCAGIPuro (Miyahara M. et. al., J. Biol. Chem. 275, 613-618(2000)) as template, primer puro5' (SEQ ID NO: 35) and primer puro3' (SEQ ID NO: 36) were used for PCR amplification of a DNA fragment containing the puromycin resistance gene (puro$^r$ gene). The DNA fragment was inserted into pT7Blue T-Vector (Novagen) to create pT7-puro.

After digesting pT7-puro with restriction enzymes (AflII and BstXI), it was inserted between the AflII and BstXI sites of the expression vector pE-neo, to create pE-puro.

With pE-puro as template, primer SV40polyA5' (SEQ ID NO: 37) and primer SV40polyA3' (SEQ ID NO: 38) were used for PCR amplification of a DNA fragment containing the SV40 late polyadenylation region. After digesting this DNA fragment with restriction enzymes (NotI and HpaI), it was inserted between the NotI and HpaI sites of the expression vector pE-puro, to create pE-puro(XhoI). After digesting pPGK-IRES-GS-$\Delta$polyA with restriction enzymes (NotI and XhoI), a DNA fragment containing the IRES-GS region was cut out and inserted between the NotI and XhoI sites of the expression vector pE-puro(XhoI), to create pE-IRES-GS-puro. Construction of pE-IRES-GS-puro was carried out with reference to patent literature (Japanese Patent No. 6279466).

[Construction of pE-mIRES-GS-puro(ΔE)]

With the expression vector pE-IRES-GS-puro as template, primer mIRES-GS5' (SEQ ID NO: 39) and primer mIRES-GS3' (SEQ ID NO: 40) were used for PCR amplification of the region from EMCV IRES to GS, and a DNA fragment disrupted by adding a mutation to the start codon (ATG) at position 2 from the 5'-end of EMCV IRES was amplified. With the expression vector pE-IRES-GS-puro as template, the DNA fragment and primer IRES5' were used for PCR amplification of a DNA fragment containing the aforementioned region from IRES to GS. The DNA fragment was digested with restriction enzymes (NotI and PstI), and the cut out DNA fragment was inserted between the NotI and PstI sites of pBluescript SK(-) (Stratagene) to create mIRES/pBlueScript SK(-).

The expression vector pE-IRES-GS-puro was digested with SphI, and the SV40 enhancer region was cut off. The remaining DNA fragment was self-ligated to create pE-IRES-GS-puro(ΔE). After digesting mIRES/pBlueScript SK(-) with NotI and PstI, the region containing modified IRES (mIRES) and part of the GS gene was cut out. Separately, pE-IRES-GS-puro(ΔE) was digested with NotI and PstI and the region containing mIRES and part of the GS gene was inserted to this, to construct pE-mIRES-GS-puro(ΔE).

[Construction of pEM-hygr(LC3) and pE-mIRES-GSp-Fab-IDUA]

A DNA fragment (CMVE-EF-1αp-IFNβMAR) containing β-Globin MAR (Matrix Attachment Region), the CMV enhancer, human EF-1α promoter, an MluI/BamHI cleavage site and interferon-β Mar was artificially synthesized (SEQ ID NO: 41). The HindIII sequence was introduced at the 5'-end and the EcoRI sequence was introduced at the 3'-end of the DNA fragment. The DNA fragment was digested with HindIII and EcoRI and inserted between the HindIII and EcoRI sites of vector pUC57 to create JCR69 in pUC57. A DNA fragment (IRES-HygroR-mPGKpA) containing the MluI/BamHI cleavage site, IRES, the hygromycin resistance gene and the mPGK polyadenylation signal was artificially synthesized (SEQ ID NO: 42). The DNA fragment was inserted at the MluI/BamHI site of JCR69 in pUC57 to create pEM hygro.

A DNA fragment (SEQ ID NO: 26) including a gene encoding the full length light chain of humanized anti-hTfR antibody having the amino acid sequence set forth as SEQ ID NO: 22 was artificially synthesized and inserted in pUC57-Amp, to create JCR131 in pUC57-Amp. The MluI sequence was introduced at the 5'-end and the NotI sequence was introduced at the 3'-end of the DNA fragment. The plasmid DNA was digested with MluI and NotI and incorporated between MluI-NotI of the expression vector pEM hygro. The obtained vector was designated as pEM-hygr(LC3), as an expression vector for the light chain of humanized anti-hTfR antibody.

A DNA fragment was artificially synthesized so as to have the nucleotide sequence set forth as SEQ ID NO: 28, containing a gene encoding a protein having as a whole the amino acid sequence set forth as SEQ ID NO: 27, wherein human IDUA having the amino acid sequence set forth as SEQ ID NO: 6 is linked to the C-terminus of the Fab region of the human anti-hTfR antibody heavy chain having the amino acid sequence set forth as SEQ ID NO: 23, via the linker sequence set forth as SEQ ID NO: 4. The MluI sequence was introduced at the 5'-end and the NotI sequence was introduced at the 3'-end of the DNA fragment. The DNA fragment was digested with MluI and NotI and incorporated between MluI and NotI of pE-mIRES-GS-puro(ΔE). The obtained vector was designated as pE-mIRES-GSp-Fab-IDUA, as an expression vector for a protein comprising hIDUA bonded to the C-terminus of the Fab heavy chain of humanized anti-hTfR antibody.

[Example 2] Creation of cell line with high expression of humanized anti-hTfR antibody-hIDUA fusion protein

CHO cells (CHO-K1: acquired from American Type Culture Collection) were transformed with pEM-hygr(LC3) and pE-mIRES-GSp-Fab-IDUA constructed in Example 1, by the following method using NEPA21 (Nepa Gene Co.).

The cell transformation was carried out generally in the following manner. The CHO-K1 cells were suspended in a 1:1 liquid mixture of CD OPTI-CHO™ medium (Thermo Fisher Scientific) and PBS, to a density of $2 \times 10^7$ cells/mL. 50 μL of cell suspension was dispensed, and to this added was 50 μL of pEM-hygr(LC3) plasmid DNA solution diluted to 200 μg/mL with a 1:1 mixture of CD OPTI-CHO™ medium and PBS. NEPA21 (Nepa Gene Co.) was used for electroporation to introduce the pEM-hygr(LC3) plasmid DNA into the cells. After culturing overnight under conditions of 37°C, 5% $CO_2$, the cells were selectively cultured in CD OPTI-CHO™ medium added with 0.5 mg/mL hygromycin. The same method was used to introduce pE-mIRES-GSp-Fab-IDUA plasmid DNA (digested with AhdI and linearized) into the obtained cells. After culturing overnight under conditions of 37°C, 5% $CO_2$, the cells were selectively cultured in CD OPTI-CHO™ medium added with 0.8 mg/mL of 0.5 mg/mL hygromycin and 10 μg/mL puromycin. After selective culturing, the MSX concentration was increased in a stepwise manner to a final MSX concentration of 300 μM, and the cells exhibiting drug resistance were selectively proliferated.

The cells were then selectively seeded by selective culturing on a 96-well plate, using a limiting dilution method so that the cells proliferated at 1 or less per well, and culturing was carried out for approximately 2 weeks with each cell forming a monoclonal colony. The culture supernatant in the wells where monoclonal colonies formed was sampled and the humanized antibody contents were examined by ELISA, and the cell lines with high expression of humanized anti-hTfR antibody-hIDUA fusion protein were selected.

The ELISA method was carried out generally in the following manner. After adding 100 μL of chicken anti-IDUA polyclonal antibody solution diluted to 5 μg/mL with 0.05 M bicarbonate buffer into each well of a 96-well microtiter plate (Nunc), it was allowed to stand for at least one hour at room temperature or 4°C for adsorption of antibody onto the plate. Each well was then washed 3 times with a mixture of 0.05% Tween20 added to Tris-buffered saline (pH 8.0) (TBS-T), a mixture of 1% BSA added to Tris-buffered saline (pH 8.0) was added at 300 μL to each well, and the plate was allowed to stand at room temperature for 1 hour. After washing each well 3 times with TBS-T, either culture supernatant or a purified product of humanized anti-hTfR antibody-hIDUA fusion protein, diluted to an appropriate concentration with Tris-buffered saline (pH 8.0) containing 0.1% BSA and 0.05% Tween20 (TBS-BT), was added at 100 μL into each well, and the plate was allowed to stand at room temperature for 1 hour. After then washing the plate 3 times with TBS-T, HRP-labeled anti-human IgG polyclonal antibody solution diluted with TBS-BT was added at 50 μL to each well, and the plate was allowed to stand at room temperature for 1 hour. After washing each well 3 times with TBS-T, an ELISA POD substrate TMB kit (Nakalai Tesque) was used for coloration. Next, 50 μL of 1 mol/L sulfuric acid was added per well to suspend the reaction, and a 96-well plate reader was used to measure the absorbance in each well at 450 nm. The cells in wells exhibiting a high measured value were selected as cell lines with high expression of humanized anti-hTfR antibody-hIDUA fusion protein. The obtained cell lines with high expression of humanized anti-hTfR antibody-hIDUA fusion protein were designated as humanized anti-hTfR antibody-hIDUA expressing lines. Fusion proteins of hIDUA and the humanized anti-hTfR antibody expressed by the cell lines were designated as humanized anti-hTfR antibody-hIDUA fusion proteins (humanized anti-hTfR antibody-hIDUA).

The obtained humanized anti-hTfR antibody-hIDUA expressing lines were suspended in CD OPTI-CHO™ medium comprising 10 mg/L insulin, 16 μmol/L thymidine, 100 μmol/L hypoxanthine, 500 μg/mL hygromycin B, 10 μg/mL puromycin, 300 μmol/L MSX and 10% (v/v) DMSO, and then dispensed into a cryotube and stored in liquid nitrogen as seed cells.

[Example 3] Culturing of humanized anti-hTfR antibody-hIDUA expressing line

A humanized anti-hTfR antibody-hIDUA expressing line was cultured by the following method to obtain humanized anti-hTfR antibody-hIDUA. A humanized anti-hTfR antibody-hIDUA expressing line obtained in Example 2 was suspended in approximately 170 L of serum-free medium (CD OPTI-CHO™ medium, ThermoFisher Scientific) adjusted to pH 6.9, comprising 10 mg/L insulin, 16 μmol/L thymidine and 100 μmol/L hypoxanthine, to a cell density of about 3 $\times$ $10^5$/mL. This 170 L of the cell suspension was transferred to a culturing tank. The medium was stirred with an impeller at a speed of about 80 to 100 rpm, and the cells were cultured for about 10 days in a temperature range of 34 to 37°C, keeping the degree of saturation of dissolved oxygen in the medium at about 30%. The cell density, cell viability, and glucose concentration and lactic acid concentration of the medium were monitored during the culturing period. When the medium glucose concentration fell below 3.0 g/L, glucose solution was immediately added to the medium to a glucose concentration of 3.5 g/L. Feed solution (EFFICIENTFEED A+™, ThermoFisher Scientific) was also added as appropriate during the culturing period. Upon completion of culturing the medium was collected. The collected medium was filtered with MILLISTAK+HC™ Pod Filter grade D0HC (Merck) and then with MILLISTAK+™ HC grade X0HC (Merck), to obtain a culture supernatant containing humanized anti-hTfR antibody-hIDUA. The culture supernatant was subjected to ultrafiltration using PELLICON™ 3 Cassette w/Ultracel PLCTK Membrane (pore size: 30 kDa, membrane area: 1.14 $m^2$, Merck), and concentrated to approximately 1/14 liquid volume. The concentrate was then filtered using OPTICAP™ XL600 (0.22 μm, Merck). The obtained solution was used as concentrated culture supernatant.

[Example 4] Purification of humanized anti-hTfR antibody-hIDUA

To the concentrated culture supernatant obtained in Example 3 a 0.25-fold volume of 2 M arginine solution (pH 7.0) was added. The solution was loaded into a CAPTURE SELECT™ CH1-XL column (column volume: about 3.1 L, bed height: about 20 cm, Thermo Fisher Scientific), which had been equilibrated with a fourfold column volume of 25 mM MES buffer (pH 6.5) containing 400 mM arginine, at a constant flow rate of 100 cm/hr, for adsorption of the humanized anti-hTfR antibody-hIDUA onto the column. The CAPTURE SELECT™ CH1-XL column is an affinity column having a carrier on which a ligand that can specifically bind to the IgG antibody CH1 domain is immobilized.

A fivefold column volume of the same buffer was then supplied at the same flow rate for washing of the column. A threefold column volume of 25 mM MES buffer solution (pH 6.5) was then supplied at the same flow rate for further washing of the column. The humanized anti-hTfR antibody-hIDUA adsorbed onto the column was eluted with a fivefold column volume of 10 mM sodium acetate-HCl buffer (pH 3.5). The eluate was received into a container already containing 250 mM MES buffer (pH 6.0), and immediately neutralized.

A 250 MM MES buffer solution (pH 6.5) was added to the eluate from the affinity column to adjust the pH of the eluate to 6.0. The eluate was then filtered using OPTICAP™ XL600 (pore size: 0.22 μm, Merck). The filtered solution was loaded into a CAPTO™ adhere column (column volume: about1.5 L, bed height: about 10 cm, GE Healthcare), a multimodal anion exchange column, which had been equilibrated with a fivefold column volume of 50 mM MES buffer solution (pH 6.0) containing 15 mM NaCl, at a constant flow rate of 300 cm/hr. The loaded solution containing humanized anti-hTfR antibody-hIDUA was collected. CAPTO™ adhere has N-benzyl-N-methylethanolamine as the ligand, and is a strong anion exchanger with selectivity based on electrostatic interaction, hydrogen bonding and hydrophobic interaction.

A fivefold column volume of the same buffer was then supplied at the same flow rate for washing of the column, and the washing solution was collected.

The loaded solution and washing solution were loaded into a CAPTO™ MMC column (column volume: ~3.1 L, bed height: ~20 cm, GE Healthcare), a multimodal weak cation exchange column, which had been equilibrated with a fourfold column volume of 25 mM MES buffer solution (pH 6.5) containing 300 mM NaCl, at a constant flow rate of 200 cm/hr. CAPTO™ MMC is a weak cation exchanger having selectivity based on hydrophobic interaction and hydrogen bond formation.

A 5 times the column volume of the same buffer was then supplied at the same flow rate for washing of the column. The humanized anti-hTfR antibody-hIDUA adsorbed onto the weak cation exchange column was then eluted with a tenfold column volume of 25 mM MES buffer solution (pH 6.5) containing 1 M NaCl.

A 0.5-fold volume of 20 mM citrate buffer solution (pH 5.5) containing 0.8 mg/mL NaCl and 75 mg/mL sucrose was added to the eluate from the weak cation exchange column, to adjust the pH to 5.8. It was then subjected to ultrafiltration using PELLICON™ 3 Cassette w/Ultracel PLCTK Membrane (pore size: 30 kDa, membrane area: 0.57 $m^2$, Merck), and concentrated to make the concentration of the humanized anti-hTfR antibody-hIDUA be approximately 30 mg/mL in the solution. The concentrate was filtered using OPTICAP™ XL600 (0.22 μm, Merck).

The concentrate was loaded into a BioSEC column (column volume: about 9.4 L, bed height: 30 cm, Merck), as a size exclusion column, which had been equilibrated with a 1.5-fold column volume of 20 mM citrate buffer solution (pH 5.5) containing 0.8 mg/mL NaCl and 75 mg/mL sucrose, at a constant flow rate of 40 cm/hr, and the same buffer was supplied at the same flow rate. An absorptiometer was disposed in the flow channel for the eluate from the size exclusion column for continuous measuring the absorbance of the eluate, the absorbance of 280 nm was monitored, and the fraction exhibiting an absorption peak at 280 nm was collected as the fraction containing humanized anti-hTfR antibody-hIDUA and used as a humanized anti-hTfR antibody-hIDUA product.

[Example 5] Examination of stability of aqueous pharmaceutical composition comprising humanized anti-hTfR antibody-hIDUA (1)

To many aqueous pharmaceutical compositions is added a nonionic surfactant in order to increase the stability of the protein as the active compound, or in order to prevent adsorption of the protein onto the container, and examples of these include aqueous pharmaceutical compositions of growth hormones, to which poloxamer 188 is added as a nonionic surfactant. The aqueous pharmaceutical composition comprising humanized anti-hTfR antibody-hIDUA was examined in the following manner to determine the effect of the addition of poloxamer 188 on protein stability.

Using the humanized anti-hTfR antibody-hIDUA product obtained in Example 4, three different aqueous pharmaceutical compositions were prepared as shown in Table 3, comprising sodium chloride, citrate buffer, sucrose, poloxamer 188 and humanized anti-hTfR antibody-hIDUA, and differing only in their concentrations of poloxamer 188. 2 mL of the three aqueous pharmaceutical compositions (formulations A to C) were each dispensed into a glass vial and sealed, and shaken by a shaking apparatus (SR-2S by Tietech Co., Ltd.) continuously for 24 hours at room temperature (shaking speed: 240 strokes/min, amplitude: 40 mm). The number of particles per unit liquid volume (200 μL) in each aqueous pharmaceutical composition after shaking (particle size: 1 to 100 μm) was measured by the method described in Example 8. The polymer content of the humanized anti-hTfR antibody-hIDUA in the aqueous pharmaceutical composition after shaking was measured by the method described in Example 9. The decomposition product content of the humanized anti-hTfR antibody-hIDUA in the aqueous pharmaceutical composition was measured by the method described in Example 10.

[Table 3]

| Table 3 Composition of aqueous pharmaceutical composition comprising humanized anti-hTfR antibody-hIDUA | | | |
|---|---|---|---|
| Component | Formulation A | Formulation B | Formulation C |
| Humanized anti-hTfR antibody-hIDUA | 5 | 5 | 5 |
| Sodium chloride | 0.8 | 0.8 | 0.8 |

(continued)

| Table 3 Composition of aqueous pharmaceutical composition comprising humanized anti-hTfR antibody-hIDUA | | | |
|---|---|---|---|
| Component | Formulation A | Formulation B | Formulation C |
| Citric acid hydrate | 1.05 | 1.05 | 1.05 |
| Sodium citrate hydrate | 4.41 | 4.41 | 4.41 |
| Sucrose | 75 | 75 | 75 |
| Poloxamer 188 | 0.25 | 0.5 | 0.8 |
| pH | 5.5 | 5.5 | 5.5 |

(Additive concentrations in mg/mL)

Fig. 1 shows measurement results for the number of particles in the aqueous pharmaceutical compositions, showing that many particles with particle sizes of less than 10 μm were present in the aqueous pharmaceutical compositions after shaking, regardless of the poloxamer 188 concentration.

Measurement results for the polymer content of the humanized anti-hTfR antibody-hIDUA in each aqueous pharmaceutical composition are shown in Fig. 2. Almost no polymer was present in the aqueous pharmaceutical compositions after shaking, regardless of the poloxamer 188 concentration.

Measurement results for the decomposition product content of the humanized anti-hTfR antibody-hIDUA in each aqueous pharmaceutical composition are shown in Fig. 3. Almost no decomposition product was present in the aqueous pharmaceutical compositions after shaking, regardless of the poloxamer 188 concentration.

[Example 6] Examination of stability of aqueous pharmaceutical composition comprising humanized anti-hTfR antibody-hIDUA (2)

An example of an aqueous pharmaceutical composition to which a nonionic surfactant is added is that to which polysorbate 80 is added as a nonionic surfactant, such as darbepoetin and agalsidase. The aqueous pharmaceutical composition comprising humanized anti-hTfR antibody-hIDUA was examined in the following manner to determine the effect of the polysorbate 80 addition on protein stability.

Using the humanized anti-hTfR antibody-hIDUA product obtained in Example 4, six different aqueous pharmaceutical compositions were prepared as shown in Table 4, comprising sodium chloride, citrate buffer, sucrose, polysorbate 80 and humanized anti-hTfR antibody-hIDUA, and differing only in their concentrations of polysorbate 80. 2 mL of the six aqueous pharmaceutical compositions (formulations D to I) were each dispensed into a glass vial and sealed, and shaken by a shaking apparatus (SR-2S by Tietech Co., Ltd.) continuously for 24 hours at room temperature (shaking speed: 240 strokes/min, amplitude: 40 mm). The number of particles per unit liquid volume (200 μL) in the aqueous pharmaceutical composition after shaking (particle size: 1 to 100 μm) was measured by the method described in Example 8. The polymer content of the humanized anti-hTfR antibody-hIDUA in the aqueous pharmaceutical composition after shaking was measured by the method described in Example 9. The decomposition product content of the humanized anti-hTfR antibody-hIDUA in the aqueous pharmaceutical composition was measured by the method described in Example 10.

[Table 4]

| Table 4 Composition of aqueous pharmaceutical composition comprising humanized anti-hTfR antibody-hIDUA | | | | | | |
|---|---|---|---|---|---|---|
| Component | Formulation D | Formulation E | Formulation F | Formulation G | Formulation H | Formulation I |
| Humanized anti-hTfR antibody-hIDUA | 5 | 5 | 5 | 5 | 5 | 5 |
| Sodium chloride | 0.8 | 0.8 | 0.8 | 0.8 | 0.8 | 0.8 |
| Citric acid hydrate | 1.05 | 1.05 | 1.05 | 1.05 | 1.05 | 1.05 |
| Sodium citrate hydrate | 4.41 | 4.41 | 4.41 | 4.41 | 4.41 | 4.41 |

(continued)

| Table 4 Composition of aqueous pharmaceutical composition comprising humanized anti-hTfR antibody-hIDUA | | | | | | |
|---|---|---|---|---|---|---|
| Component | Formulation D | Formulation E | Formulation F | Formulation G | Formulation H | Formulation I |
| Sucrose | 75 | 75 | 75 | 75 | 75 | 75 |
| Polysorbate 80 | 0.025 | 0.05 | 0.1 | 0.25 | 0.5 | 0.8 |
| pH | 5.5 | 5.5 | 5.5 | 5.5 | 5.5 | 5.5 |

(Additive concentrations in mg/mL)

Fig. 4 shows measurement results for number of particles in an aqueous pharmaceutical composition. When the polysorbate 80 concentration was 0.25 mg/mL or greater, the number of particles with particle sizes of less than 10 μm in the aqueous pharmaceutical composition after shaking was markedly smaller compared to when the polysorbate 80 concentration was 0.1 mg/mL or lower.

Measurement results for the polymer content of the humanized anti-hTfR antibody-hIDUA in each aqueous pharmaceutical composition are shown in Fig. 5. A higher polysorbate 80 concentration resulted in a lower amount of polymer in the aqueous pharmaceutical composition after shaking, with almost no polymer found in the aqueous pharmaceutical composition when the polysorbate 80 concentration was 0.5 mg/mL or higher.

Measurement results for the decomposition product content of the humanized anti-hTfR antibody-hIDUA in each aqueous pharmaceutical composition are shown in Fig. 6. Almost no decomposition product was found in the aqueous pharmaceutical compositions after shaking, regardless of the polysorbate 80 concentration.

Based on Example 5 and these results, addition of poloxamer 188 is effective for inhibiting generation of humanized anti-hTfR antibody-hIDUA polymer in the aqueous pharmaceutical composition, while addition of polysorbate 80 is effective for inhibiting increase in microparticles with particle sizes of less than 10 μm, or in other words, each surfactant was concluded to have a different effect on stability of the aqueous pharmaceutical composition.

[Example 7] Examination of stability of aqueous pharmaceutical composition comprising humanized anti-hTfR antibody-hIDUA (3)

Using the humanized anti-hTfR antibody-hIDUA product obtained in Example 4, five different aqueous pharmaceutical compositions were prepared as shown in Table 5, comprising sodium chloride, citrate buffer, sucrose, poloxamer 188, polysorbate 80 and humanized anti-hTfR antibody-hIDUA, and differing only in their concentrations of polysorbate 80. 2 mL of the five aqueous pharmaceutical compositions (formulations J to N) were each dispensed into a glass vial and sealed, and shaken by a shaking apparatus (SR-2S by Tietech Co., Ltd.) continuously for 24 hours at room temperature (shaking speed: 240 strokes/min, amplitude: 40 mm). The number of particles per unit liquid volume (200 μL) in the aqueous pharmaceutical composition after shaking (particle size: 1 to 100 μm) was measured by the method described in Example 8. The polymer content of the humanized anti-hTfR antibody-hIDUA in the aqueous pharmaceutical composition after shaking was measured by the method described in Example 9. The decomposition product content of the humanized anti-hTfR antibody-hIDUA in the aqueous pharmaceutical composition was measured by the method described in Example 10.

[Table 5]

| Table 5 Composition of aqueous pharmaceutical composition comprising humanized anti-hTfR antibody-hIDUA | | | | | |
|---|---|---|---|---|---|
| Component | Formulation J | Formulation K | Formulation L | Formulation M | Formulation N |
| Humanized anti-hTfR antibody-hIDUA | 5 | 5 | 5 | 5 | 5 |
| Sodium chloride | 0.8 | 0.8 | 0.8 | 0.8 | 0.8 |
| Citric acid hydrate | 1.05 | 1.05 | 1.05 | 1.05 | 1.05 |
| Sodium citrate hydrate | 4.41 | 4.41 | 4.41 | 4.41 | 4.41 |
| Sucrose | 75 | 75 | 75 | 75 | 75 |

(continued)

| Table 5 Composition of aqueous pharmaceutical composition comprising humanized anti-hTfR antibody-hIDUA | | | | | |
|---|---|---|---|---|---|
| Component | Formulation J | Formulation K | Formulation L | Formulation M | Formulation N |
| Poloxamer 188 | 0.325 | 0.325 | 0.325 | 0.325 | 0.325 |
| Polysorbate 80 | 0.025 | 0.05 | 0.1 | 0.25 | 0.5 |
| pH | 5.5 | 5.5 | 5.5 | 5.5 | 5.5 |

(Additive concentrations in mg/mL)

Fig. 7 shows measurement results for number of particles in an aqueous pharmaceutical composition. When the polysorbate 80 concentration was 0.05 mg/mL or greater, the number of particles with particle sizes of less than 10 μm in the aqueous pharmaceutical composition after shaking was smaller compared to when the polysorbate 80 concentration was 0.025 mg/mL or lower.

Measurement results for the polymer content of the humanized anti-hTfR antibody-hIDUA in each aqueous pharmaceutical composition are shown in Fig. 8. Almost no polymer was found in the aqueous pharmaceutical compositions, regardless of the polysorbate 80 concentration.

Measurement results for the decomposition product content of the humanized anti-hTfR antibody-hIDUA in each aqueous pharmaceutical composition are shown in Fig. 9. Almost no decomposition product was found in the aqueous pharmaceutical compositions after shaking, regardless of the polysorbate 80 concentration.

Based on the results, addition of both poloxamer 188 and polysorbate 80 is effective for simultaneously inhibiting both generation of humanized anti-hTfR antibody-hIDUA polymer and increase in microparticles with particle sizes of less than 10 μm in the aqueous pharmaceutical composition, while addition of polysorbate 80 is effective for inhibiting increase in microparticles with particle sizes of less than 10 μm. However, since the aqueous pharmaceutical composition is to be administered as a medicine to a human, it is preferred for the poloxamer 188 and polysorbate 80 concentrations to be low. For example, a stable aqueous pharmaceutical composition can be produced by limiting the poloxamer 188 and polysorbate 80 concentrations in the aqueous pharmaceutical composition to the ranges of 0.15 to 0.5 mg/mL and 0.025 to 0.125 mg/mL, respectively.

[Example 8] Measurement of number of particles in aqueous pharmaceutical composition (particle size: 1 to 100 μm)

The number of particles in the aqueous pharmaceutical composition was measured using the flow imaging particle analyzer FLOWCAM™ (Fluid Imaging Technologies). A flow imaging particle analyzer is an apparatus that measures the number of particles in a sample solution by using a syringe pump to suction the sample solution into a flow cell perpendicular to an optical system, and photographing the particles passing through the flow cell in real time. The measurement was carried out with the detection particle size set to 1 to 100 μm.

[Example 9] Measurement of polymer content of humanized anti-hTfR antibody-hIDUA in aqueous pharmaceutical composition

A 5 μm TSKgel G3000SW$_{XL}$ size exclusion column chromatography column (7.8 mm diameter × 30 cm length, Tosoh Co.) was set in a Shimadzu LC-20A HPLC system (Shimadzu Corp.). An absorptiometer was set downstream from the column, allowing continuous measurement of absorbance (measuring wavelength: 215 nm) of the effluent from the column. After equilibrating the column with 0.2 M aqueous sodium phosphate buffer, a sample solution comprising 10 μg of humanized anti-hTfR antibody-hIDUA was loaded into the column, and additional 0.2 M aqueous sodium phosphate buffer was flowed through at a flow rate of 0.6 mL/min. An elution profile was obtained by measuring the absorbance (measuring wavelength: 215 nm) of the effluent from the column during this time. The obtained elution profile was used to determine the peak area of the humanized anti-hTfR antibody-hIDUA monomer (monomer peak area), the peak area of the humanized anti-hTfR antibody-hIDUA polymer (polymer peak area) appearing before the monomer peak, and the peak area of the humanized anti-hTfR antibody-hIDUA decomposition product (decomposition product peak area) appearing after the monomer peak. The polymer content (%) was calculated by the following formula.

$$\text{Polymer content (\%)} = \{\text{Polymer peak area}/(\text{monomer peak area} + \text{polymer peak area} + \text{decomposition product peak area})\} \times 100$$

[Example 10] Measurement of decomposition product content of humanized anti-hTfR antibody-hIDUA in aqueous pharmaceutical composition

A 5 μm TSKgel G3000$SW_{XL}$ size exclusion column chromatography column (7.8 mm diameter × 30 cm length, Tosoh Co.) was set in a Shimadzu LC-20A HPLC system (Shimadzu Corp.). An absorptiometer was set downstream from the column, allowing continuous measurement of absorbance (measuring wavelength: 215 nm) of the effluent from the column. After equilibrating the column with 0.2 M aqueous sodium phosphate buffer, a sample solution comprising 10 μg of humanized anti-hTfR antibody-hIDUA was loaded into the column, and additional 0.2 M aqueous sodium phosphate buffer was flowed through at a flow rate of 0.6 mL/min. An elution profile was obtained by measuring the absorbance (measuring wavelength: 215 nm) of the effluent from the column during this time. The obtained elution profile was used to determine the peak area of the humanized anti-hTfR antibody-hIDUA monomer (monomer peak area), the peak area of the humanized anti-hTfR antibody-hIDUA polymer (polymer peak area) appearing before the monomer peak, and the peak area of the humanized anti-hTfR antibody-hIDUA decomposition product (decomposition product peak area) appearing after the monomer peak. The decomposition product content (%) was calculated by the following formula.

$$\text{Decomposition product content (\%)} = \{\text{Decomposition product peak area}/(\text{monomer peak area} + \text{polymer peak area} + \text{decomposition product peak area})\} \times 100$$

[Example 11] Aqueous pharmaceutical composition formulation design

A Formulation Example for an aqueous pharmaceutical composition comprising humanized anti-hTfR antibody-hIDUA was designed based on the results of Examples 5 to 7, using the composition shown in Table 6 (Formulation O). The aqueous pharmaceutical composition was filled or encapsulated in a glass or plastic vial, ampule or syringe at a liquid volume of 1 to 10 mL, and stored at low temperature (4°C, for example). The product filled or encapsulated in a syringe is as a prefilled syringe-type formulation.

[Table 6]

Table 6 Composition of aqueous pharmaceutical composition comprising humanized anti-hTfR antibody-hIDUA

| Component | Formulation O |
|---|---|
| Humanized anti-hTfR antibody-hIDUA | 5 |
| Sodium chloride | 0.8 |
| Citric acid hydrate | 1.05 |
| Sodium citrate hydrate | 4.41 |
| Sucrose | 75 |
| Poloxamer 188 | 0.325 |
| Polysorbate 80 | 0.075 |
| pH | 5.5 |

(Additive concentrations in mg/mL)

[Example 12] Prolonged storage test for aqueous pharmaceutical composition

2 mL of Formulation O, as an aqueous pharmaceutical composition comprising humanized anti-hTfR antibody-hIDUA, was filled into a glass vial and stored in a dark environment at a temperature of 2 to 8°C. During the storage period, the solution pH, the number of particles per unit liquid volume (200 μL) (particle size: 1 to 100 μm), the polymer content of the humanized anti-hTfR antibody-hIDUA and the decomposition product content of the humanized anti-hTfR antibody-hIDUA were periodically measured. The number of particles was measured by the method described in Example 8, the humanized anti-hTfR antibody-hIDUA polymer content was measured by the method described in Example 9, and the humanized anti-hTfR antibody-hIDUA decomposition product content was measured by the method described in Example 10.

Table 7 shows the results of a prolonged storage test for Formulation O of the aqueous pharmaceutical composition comprising humanized anti-hTfR antibody-hIDUA. The solution pH, the number of particles per unit liquid volume (200 μL) (particle size: 1 to 100 μm), the humanized anti-hTfR antibody-hIDUA polymer content, and the humanized anti-hTfR antibody-hIDUA decomposition product content were measured at the start of storage and 1 month, 2 months, 3 months, 6 months, 9 months, 12 months, 18 months, 24 months and 36 months after the start of storage. Virtually no change was observed in the pH, number of particles, polymer (%) or decomposition product (%) during the storage period of the prolonged storage test. These results indicate that Formulation O of the aqueous pharmaceutical composition comprising humanized anti-hTfR antibody-hIDUA is stable for at least 36 months in a dark environment at a temperature of 2 to 8°C, and it is predicted to be stable even 72 months after the start of storage.

[Table 7]

| Table 7 Results of long-term storage test for aqueous pharmaceutical formulation O comprising humanized anti-hTfR antibody-hIDUA | | | | | |
|---|---|---|---|---|---|
| Time point | pH | Number of particles (particle size <10 μm) | Number of particles (particle size ≥10 μm) | Polymer (%) | Decomposition product (%) |
| Initial storage | 5.51 | 859 | 69 | 0.16 | 0.11 |
| 1 month | 5.50 | 137 | 11 | 0.20 | 0.12 |
| 2 months | 5.50 | 736 | 6 | 0.25 | 0.14 |
| 3 months | 5.50 | 257 | 6 | 0.23 | 0.15 |
| 6 months | 5.50 | 1877 | 40 | 0.19 | 0.20 |
| 9 months | 5.51 | 20720 | 287 | 0.19 | 0.27 |
| 12 months | 5.50 | 7667 | 97 | 0.17 | 0.26 |
| 18 months | 5.48 | 509 | 0 | 0.21 | 0.37 |
| 24 months | 5.48 | 3564 | 58 | 0.17 | 0.43 |
| 36 months | 5.50 | 1785 | 53 | 0.24 | 0.56 |

[Example 13] Production of lyophilized pharmaceutical composition

2.4 mL of Formulation O of the aqueous pharmaceutical composition comprising humanized anti-hTfR antibody-hIDUA was filled into a glass vial, half-plugged with a (chlorobutyl) rubber stopper, and lyophilized. During the lyophilization step, the rubber stopper was fully plugged to seal the vial after exchanging the gas phase of the vial to nitrogen. The lyophilized product formed a white mass in the vial. The obtained lyophilized product can be restored to the original aqueous pharmaceutical composition by adding purified water to the vial and shaking the vial to form a 2.4 mL solution.

[Example 14] Prolonged storage test for lyophilized pharmaceutical composition

The Formulation O obtained in Example 13, as a lyophilized pharmaceutical composition comprising humanized anti-hTfR antibody-hIDUA, was stored in a dark environment at a temperature of 2 to 8°C. During the storage period, the pH, the number of particles per unit liquid volume (200 μL) (particle size: 1 to 100 μm), the humanized anti-hTfR antibody-hIDUA polymer content and the humanized anti-hTfR antibody-hIDUA decomposition product content of a solution obtained by dissolving the lyophilized pharmaceutical composition in purified water were periodically measured. The number of particles was measured by the method described in Example 8, the humanized anti-hTfR antibody-hIDUA polymer content was measured by the method described in Example 9, and the humanized anti-hTfR antibody-hIDUA decomposition product content was measured by the method described in Example 10.

Table 8 shows the results of a prolonged storage test for a lyophilized pharmaceutical composition of Formulation O, comprising humanized anti-hTfR antibody-hIDUA. The solution pH, the number of particles per unit liquid volume (200 μL) (particle size: 1 to 100 μm), the humanized anti-hTfR antibody-hIDUA polymer content, and the humanized anti-hTfR antibody-hIDUA decomposition product content were measured at the start of storage and 1 month, 2 months, 3 months, 6 months, 9 months, 12 months, 18 months, 24 months and 36 months after the start of storage. Virtually no change was observed in the pH, number of particles, polymer (%) or decomposition product (%) during the storage period of the prolonged storage test. These results indicate that Formulation O in the lyophilized pharmaceutical composition comprising humanized anti-hTfR antibody-hIDUA is stable for at least 36 months in a dark environment at a temperature of 2 to 8°C, and it is predicted to be stable even 72 months after the start of storage.

[Table 8]

Table 8 Results of long-term storage test for lyophilized pharmaceutical formulation O comprising humanized anti-hTfR antibody-hIDUA

| Time point | pH | Number of particles (particle size <10 μm) | Number of particles (particle size ≥10 μm) | Polymer (%) | Decomposition product (%) |
|---|---|---|---|---|---|
| Initial storage | 5.51 | 561 | 6 | 0.16 | 0.10 |
| 1 month | 5.51 | 166 | 0 | 0.20 | 0.10 |
| 2 months | 5.51 | 1280 | 34 | 0.24 | 0.11 |
| 3 months | 5.50 | 355 | 0 | 0.20 | 0.10 |
| 6 months | 5.50 | 1923 | 40 | 0.17 | 0.11 |
| 9 months | 5.51 | 6553 | 80 | 0.18 | 0.15 |
| 12 months | 5.50 | 755 | 11 | 0.15 | 0.09 |
| 18 months | 5.49 | 316 | 18 | 0.16 | 0.06 |
| 24 months | 5.48 | 807 | 6 | 0.16 | 0.05 |
| 36 months | 5.41 | 533 | 0 | 0.22 | 0.03 |

**Industrial Applicability**

According to the invention it is possible to commercially provide an aqueous pharmaceutical composition or lyophilized pharmaceutical composition that comprises a protein having physiological activity as an active ingredient.

[Sequence Listing Free Text]

SEQ ID NO: 1: Amino acid sequence of linker example 1
SEQ ID NO: 2: Amino acid sequence of linker example 2
SEQ ID NO: 3: Amino acid sequence of linker example 3
SEQ ID NO: 4: Amino acid sequence of linker example 4
SEQ ID NO: 5: Amino acid sequence of human transferrin receptor
SEQ ID NO: 6: Amino acid sequence of human IDUA (1)
SEQ ID NO: 7: Amino acid sequence of human IDUA (2)
SEQ ID NO: 8: Amino acid sequence of anti-hTfR antibody light chain CDR1 (1)
SEQ ID NO: 9: Amino acid sequence of anti-hTfR antibody light chain CDR1 (2)
SEQ ID NO: 10: Amino acid sequence of anti-hTfR antibody light chain CDR2 (1)
SEQ ID NO: 11: Amino acid sequence of anti-hTfR antibody light chain CDR2 (2)
SEQ ID NO: 12: Amino acid sequence of anti-hTfR antibody light chain CDR3 (1)
SEQ ID NO: 13: Amino acid sequence of anti-hTfR antibody heavy chain CDR1 (1)
SEQ ID NO: 14: Amino acid sequence of anti-hTfR antibody heavy chain CDR1 (2)
SEQ ID NO: 15: Amino acid sequence of anti-hTfR antibody heavy chain CDR2 (1)
SEQ ID NO: 16: Amino acid sequence of anti-hTfR antibody heavy chain CDR2 (2)
SEQ ID NO: 17: Amino acid sequence of anti-hTfR antibody heavy chain CDR3 (1)
SEQ ID NO: 18: Amino acid sequence of anti-hTfR antibody heavy chain CDR3 (2)
SEQ ID NO: 19: Amino acid sequence of anti-hTfR antibody heavy chain framework region (3)
SEQ ID NO: 20: Amino acid sequence of anti-hTfR antibody light chain variable region
SEQ ID NO: 21: Amino acid sequence of anti-hTfR antibody heavy chain variable region

SEQ ID NO: 22: Amino acid sequence of anti-hTfR antibody light chain
SEQ ID NO: 23: Amino acid sequence of anti-hTfR antibody Fab heavy chain
SEQ ID NO: 24: Primer Hyg-Sfi5', synthetic sequence
SEQ ID NO: 25: Primer Hyg-BstX3', synthetic sequence
SEQ ID NO: 26: Nucleotide sequence encoding amino acid sequence of anti-hTfR antibody light chain, synthetic sequence
SEQ ID NO: 27: Amino acid sequence of fusion protein of humanized anti-hTfR antibody Fab heavy chain and human IDUA
SEQ ID NO: 28: Nucleotide sequence of gene encoding amino acid sequence of fusion protein of humanized anti-hTfR antibody Fab heavy chain and human IDUA, synthetic sequence
SEQ ID NO: 29: Primer IRES5', synthetic sequence
SEQ ID NO: 30: Primer IRES3', synthetic sequence
SEQ ID NO: 31: Primer mPGKP5', synthetic sequence
SEQ ID NO: 32: Primer mPGKP3', synthetic sequence
SEQ ID NO: 33: Primer GS5', synthetic sequence
SEQ ID NO: 34: Primer GS3', synthetic sequence
SEQ ID NO: 35: Primer puro5', synthetic sequence
SEQ ID NO: 36: Primer puro3', synthetic sequence
SEQ ID NO: 37: Primer SV40polyA5', synthetic sequence
SEQ ID NO: 38: Primer SV40polyA3', synthetic sequence
SEQ ID NO: 39: Primer mIRES-GS5', synthetic sequence
SEQ ID NO: 40: Primer mIRES-GS3', synthetic sequence
SEQ ID NO: 41: CMVE-EF-1αp-IFNβMAR, synthetic sequence
SEQ ID NO: 42: IRES-HygroR-mPGKpA, synthetic sequence

**Claims**

1. An aqueous pharmaceutical composition or lyophilized pharmaceutical composition comprising a protein having physiological activity, and two different nonionic surfactants.

2. The aqueous pharmaceutical composition or lyophilized pharmaceutical composition according to claim 1 further comprising one or more of a neutral salt, a disaccharide, and a buffering agent.

3. The aqueous pharmaceutical composition or lyophilized pharmaceutical composition according to claim 1 or 2, which includes a polysorbate and poloxamer as the nonionic surfactants.

4. The aqueous pharmaceutical composition or lyophilized pharmaceutical composition according to claim 3, wherein:

   the polysorbate is polysorbate 20 or polysorbate 80, and
   the poloxamer is selected from the group consisting of polyoxyethylene(42) polyoxypropylene(67) glycol, polyoxyethylene(54) polyoxypropylene(39) glycol, polyoxyethylene(196) polyoxypropylene(67) glycol, polyoxyethylene(42) polyoxypropylene(67) glycol, polyoxyethylene(3) polyoxypropylene(17) glycol, polyoxyethylene(20) polyoxypropylene(20) glycol and polyoxyethylene (120) polyoxypropylene(40) glycol.

5. The aqueous pharmaceutical composition or lyophilized pharmaceutical composition according to claim 3, wherein the polysorbate is polysorbate 80 and the poloxamer is polyoxyethylene(160) polyoxypropylene(30) glycol.

6. The aqueous pharmaceutical composition according to any one of claims 3 to 5, wherein the concentration of the polysorbate is 0.005 to 1.5 mg/mL and the concentration of the poloxamer is 0.05 to 0.6 mg/mL.

7. The aqueous pharmaceutical composition according to any one of claims 3 to 5, wherein the concentration of the polysorbate is 0.025 to 1.0 mg/mL and the concentration of the poloxamer is 0.1 to 0.5 mg/mL.

8. The aqueous pharmaceutical composition according to any one of claims 3 to 5, wherein the concentration of the polysorbate is 0.05 to 0.15 mg/mL and the concentration of the poloxamer is 0.15 to 0.45 mg/mL.

9. The aqueous pharmaceutical composition according to any one of claims 1 to 8, wherein the neutral salt is sodium chloride.

**10.** The aqueous pharmaceutical composition according to any one of claims 1 to 9, wherein the disaccharide is selected from the group consisting of trehalose, sucrose, maltose, lactose and combinations of two or more of the same.

**11.** The aqueous pharmaceutical composition according to any one of claims 1 to 10, wherein the buffering agent is selected from the group consisting of citrate buffer, phosphate buffer, glycine buffer, histidine buffer, carbonate buffer, acetate buffer, and combinations of two or more of the foregoing.

**12.** The aqueous pharmaceutical composition according to any one of claims 3 to 5, wherein the concentration of the neutral salt is 0.3 to 1.2 mg/mL, the concentration of the disaccharide is 50 to 100 mg/mL, the concentration of the buffering agent is 10 to 30 mM, the concentration of the polysorbate is 0.005 to 1.5 mg/mL and the concentration of the poloxamer is 0.1 to 0.6 mg/mL.

**13.** The aqueous pharmaceutical composition according to any one of claims 3 to 5, wherein the concentration of the neutral salt is 0.5 to 1.0 mg/mL, the concentration of the disaccharide is 55 to 95 mg/mL, the concentration of the buffering agent is 15 to 25 mM, the concentration of the polysorbate is 0.05 to 1.0 mg/mL and the concentration of the poloxamer is 0.25 to 0.45 mg/mL.

**14.** The aqueous pharmaceutical composition according to any one of claims 3 to 5, wherein the concentration of the neutral salt is 0.7 to 0.9 mg/mL, the concentration of the disaccharide is 60 to 90 mg/mL, the concentration of the buffering agent is 15 to 25 mM, the concentration of the polysorbate is 0.05 to 0.15 mg/mL and the concentration of the poloxamer is 0.25 to 0.45 mg/mL.

**15.** The aqueous pharmaceutical composition according to any one of claims 1 to 14, wherein the pH is 4.5 to 6.5.

**16.** The aqueous pharmaceutical composition according to any one of claims 1 to 14, wherein the pH is 5.0 to 6.0.

**17.** The aqueous pharmaceutical composition according to any one of claims 1 to 14, wherein the pH is 5.2 to 5.8.

**18.** The aqueous pharmaceutical composition according to any one of claims 1 to 17, wherein the protein having physiological activity is the fusion protein of an antibody and a lysosomal enzyme.

**19.** The aqueous pharmaceutical composition according to claim 18, wherein the fusion protein is the lysosomal enzyme bonded by a peptide bond at either the C-terminus or N-terminus of either the antibody light chain or heavy chain.

**20.** The aqueous pharmaceutical composition according to claim 18, wherein the fusion protein is the lysosomal enzyme bonded by a peptide bond at the C-terminus of the antibody heavy chain.

**21.** The aqueous pharmaceutical composition according to claim 18, wherein the fusion protein is the lysosomal enzyme bonded at either the C-terminus or N-terminus of either the antibody light chain or heavy chain via a linker consisting of at least one amino acid.

**22.** The aqueous pharmaceutical composition according to claim 18, wherein the fusion protein is the lysosomal enzyme bonded at the C-terminus of the antibody heavy chain via a linker consisting of at least one amino acid.

**23.** The aqueous pharmaceutical composition according to claim 21 or 22, wherein the linker comprises an amino acid sequence selected from the group consisting of Gly-Ser, Gly-Gly-Ser, SEQ ID NO: 1, SEQ ID NO: 2, SEQ ID NO: 3, SEQ ID NO: 4, and 2 to 10 of any of aforementioned amino acid sequences that are consecutively linked.

**24.** The aqueous pharmaceutical composition according to any one of claims 18 to 23, wherein the lysosomal enzyme is a human lysosomal enzyme.

**25.** The aqueous pharmaceutical composition according to any one of claims 18 to 24, wherein the lysosomal enzyme is selected from the group consisting of α-L-iduronidase, iduronate-2-sulfatase, glucocerebrosidase, β-galactosidase, GM2 activated protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase, β-glucuronidase, heparan N-sulfatase, α-N-acetylglucosaminidase, acetyl CoAα-glucosaminide N-acetyltransferase, N-acetylglucosamine-6-sulfatase, acid ceramidase, amylo-1,6-glucosidase, sialidase, aspartylglucosaminidase, palmitoyl protein thioesterase-1, tripeptidyl peptidase-1, hyaluronidase-1, CLN1 and CLN2.

**26.** The aqueous pharmaceutical composition according to claim 24, wherein the human lysosomal enzyme is α-L-iduronidase.

**27.** The aqueous pharmaceutical composition according to any one of claims 18 to 26, wherein the antibody is a human antibody or humanized antibody.

**28.** The aqueous pharmaceutical composition according to any one of claims 18 to 27, wherein the antibody is a Fab antibody, $F(ab')_2$ antibody or F(ab') antibody.

**29.** The aqueous pharmaceutical composition according to any one of claims 18 to 28, wherein the antibody recognizes as antigen a molecule present on the surfaces of vascular endothelial cells.

**30.** The aqueous pharmaceutical composition according to claim 29, wherein the vascular endothelial cells are human vascular endothelial cells.

**31.** The aqueous pharmaceutical composition according to claim 29 or 30, wherein the vascular endothelial cells are cerebrovascular endothelial cells.

**32.** The aqueous pharmaceutical composition according to claim 31, wherein the molecule present on the surfaces of cerebrovascular endothelial cells is selected from the group consisting of transferrin receptor (TfR), insulin receptor, leptin receptor, lipoprotein receptor, IGF receptor, OATP-F, organic anion transporter and monocarboxylate transporter.

**33.** The aqueous pharmaceutical composition according to claim 28, wherein the antibody is a humanized anti-human transferrin receptor (hTfR) antibody.

**34.** The aqueous pharmaceutical composition according to claim 28, wherein the antibody is the Fab antibody of humanized anti-human transferrin receptor (hTfR) antibody, the human lysosomal enzyme is human α-L-iduronidase, the fusion protein is a fusion protein of the antibody and the human α-L-iduronidase, and in the fusion protein:
the antibody light chain includes the amino acid sequence set forth as SEQ ID NO: 22, and
the antibody heavy chain is bonded at the C-terminus with human α-L-iduronidase via the amino acid sequence set forth as SEQ ID NO: 4, thereby forming the amino acid sequence set forth as SEQ ID NO: 27.

**35.** The aqueous pharmaceutical composition according to claim 28, wherein the antibody is the Fab antibody of humanized anti-human transferrin receptor (hTfR) antibody, the human lysosomal enzyme is human α-L-iduronidase, the fusion protein is a fusion protein of the antibody and the human α-L-iduronidase, and in the fusion protein:
the antibody light chain includes the amino acid sequence set forth as SEQ ID NO: 22, and
the antibody heavy chain includes the amino acid sequence set forth as SEQ ID NO: 23, the heavy chain being bonded at the C-terminus with human α-L-iduronidase having the amino acid sequence set forth as SEQ ID NO: 5 or SEQ ID NO: 6, via the amino acid sequence set forth as SEQ ID NO: 4.

**36.** The aqueous pharmaceutical composition according to any one of claims 1 to 35, which is encapsulated in a container formed of borosilicate glass or a hydrophobic resin.

**37.** The aqueous pharmaceutical composition according to claim 36, wherein the container is formed of a cycloolefin copolymer, a cycloolefin ring-opening polymer or a hydrogenated cycloolefin ring-opening polymer.

**38.** A lyophilized pharmaceutical composition obtained by lyophilizing the aqueous pharmaceutical composition according to any one of claims 6 to 35.

**39.** The lyophilized pharmaceutical composition according to claim 38, which is encapsulated in a container whose material includes borosilicate glass or a hydrophobic resin.

**40.** The lyophilized pharmaceutical composition according to claim 39, wherein the material of the container includes a cycloolefin copolymer, a cycloolefin ring-opening polymer or a hydrogenated cycloolefin ring-opening polymer.

**41.** The aqueous pharmaceutical composition or lyophilized pharmaceutical composition according to any one of claims 1 to 40, wherein the content ratio of the polymer after storage for 36 months in a dark environment at a temperature of 2 to 8°C is 0.5% or lower.

**42.** The aqueous pharmaceutical composition according to any one of claims 1 to 37, wherein the content ratio of the polymer and the content ratio of decomposition products after storage for 36 months in a dark environment at a temperature of 2 to 8°C are 0.5% or lower and 1% or lower, respectively.

**43.** The lyophilized pharmaceutical composition according to any one of claims 39 to 41, wherein the content ratio of the polymer and the content ratio of decomposition products after storage for 36 months in a dark environment at a temperature of 2 to 8°C are 0.5% or lower and 0.1% or lower, respectively.

Fig.1

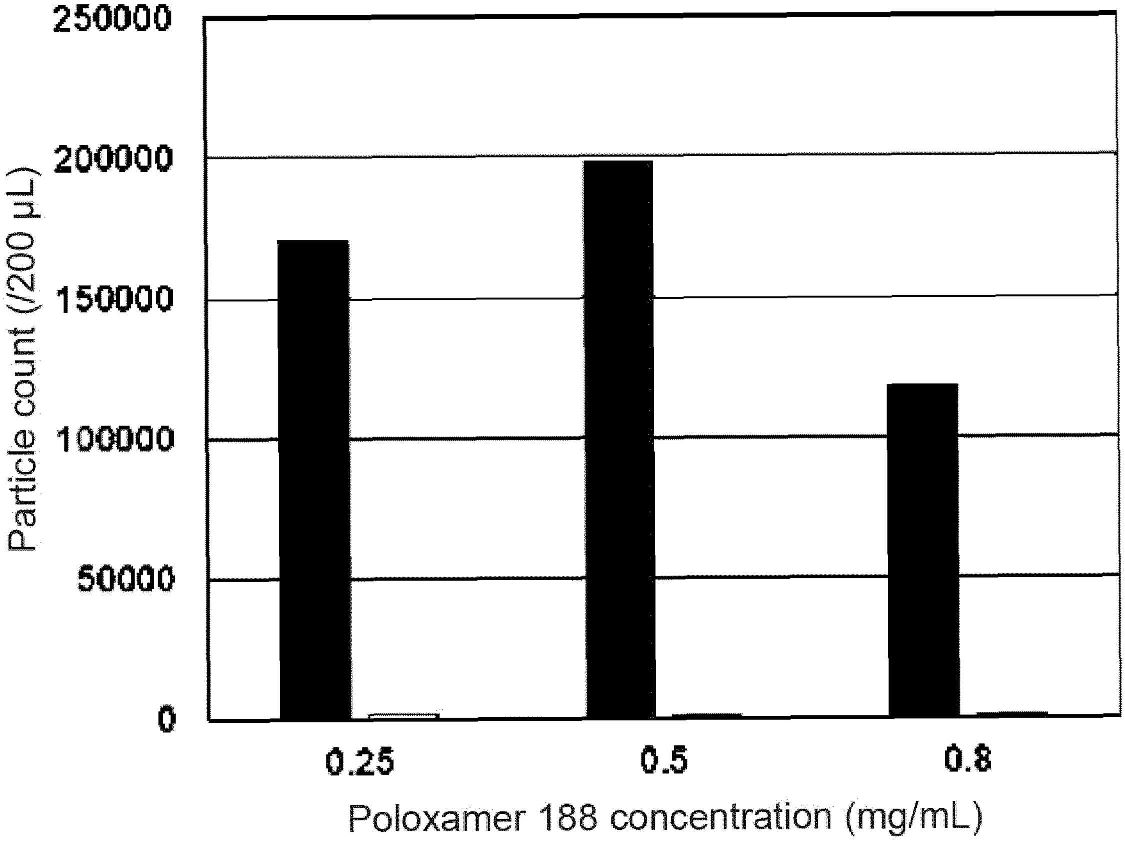
250000
200000
150000
100000
50000
0
Particle count (/200 µL)
0.25
0.5
0.8
Poloxamer 188 concentration (mg/mL)

*Fig.2*

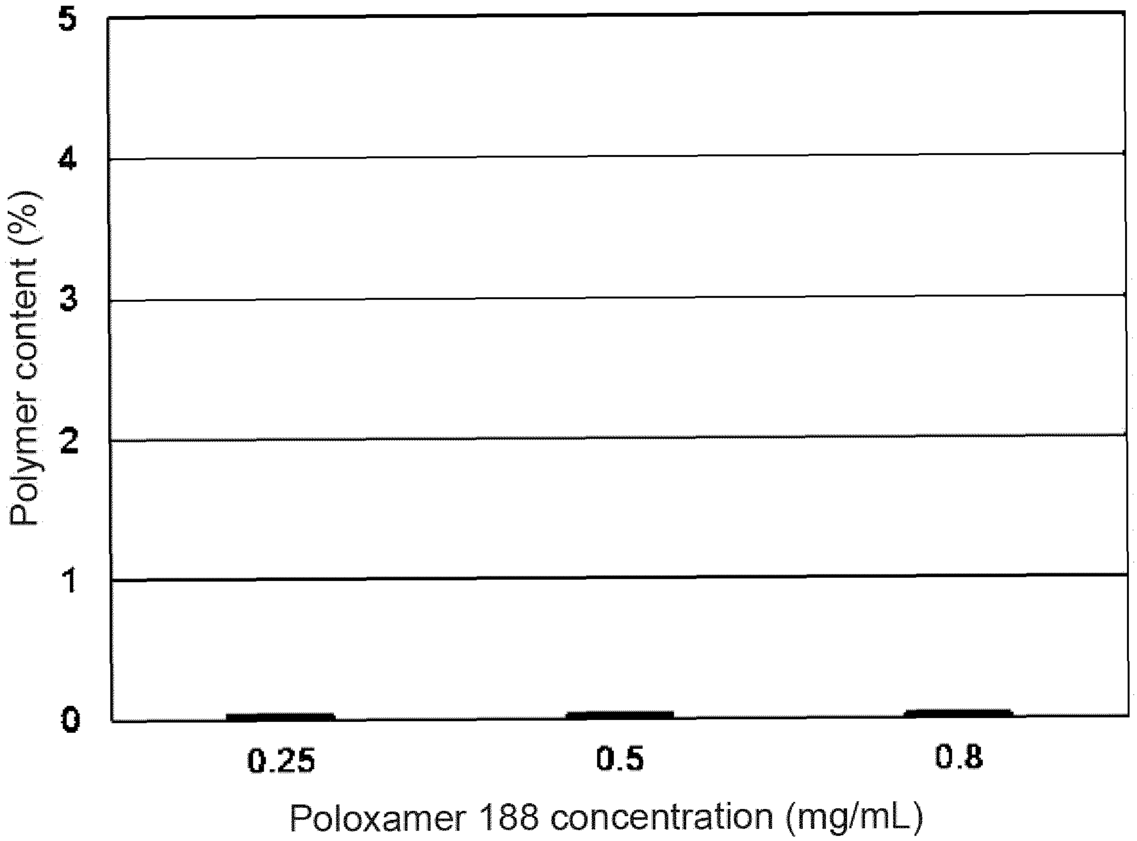
5
4
3
2
1
0
Polymer content (%)
0.25
0.5
0.8
Poloxamer 188 concentration (mg/mL)

*Fig.3*

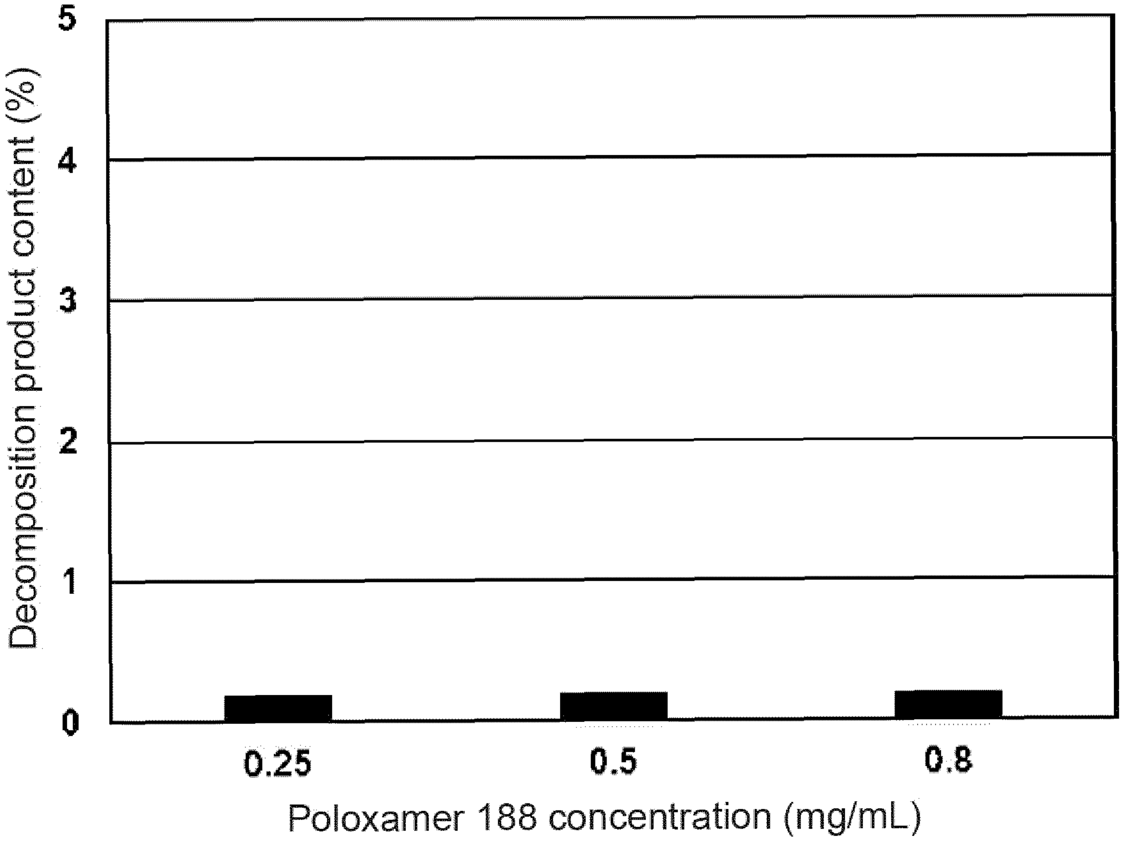
5
4
3
2
1
0
Decomposition product content (%)
0.25
0.5
0.8
Poloxamer 188 concentration (mg/mL)

Fig.4

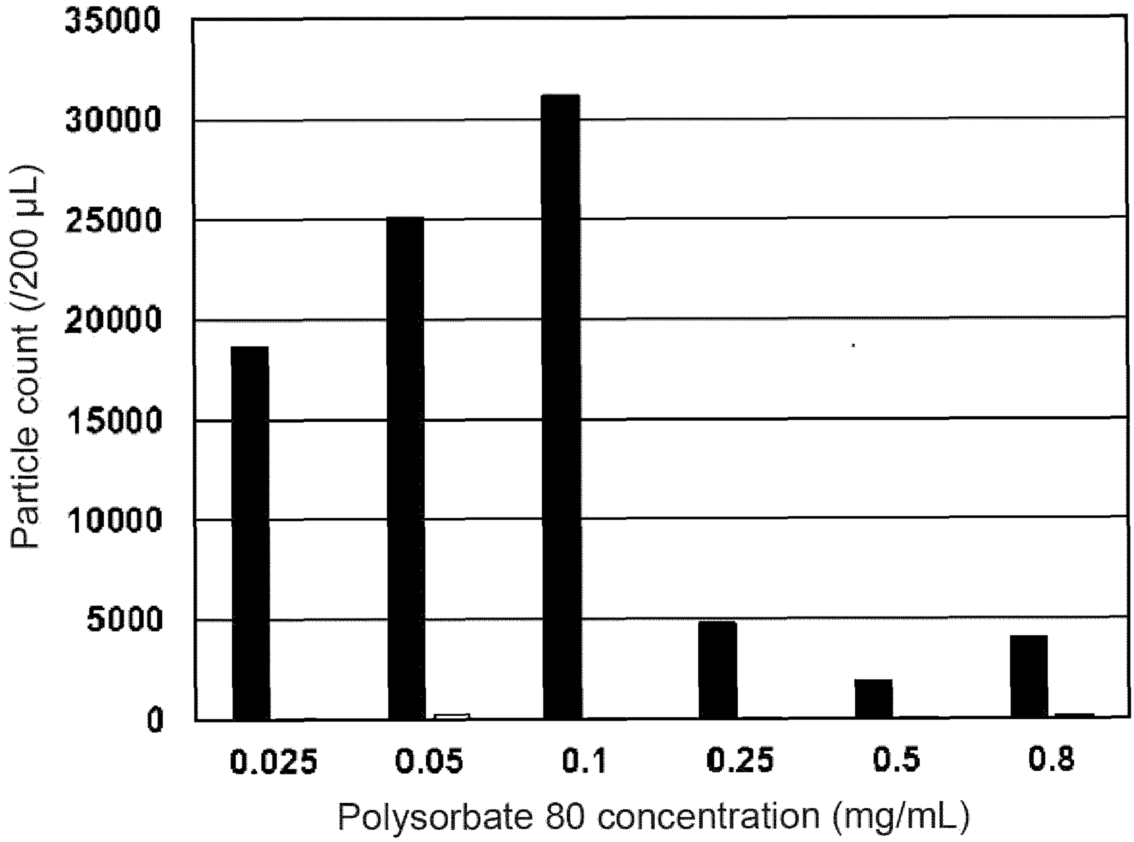
35000
30000
25000
20000
15000
10000
5000
0
Particle count (/200 μL)
0.025
0.05
0.1
0.25
0.5
0.8
Polysorbate 80 concentration (mg/mL)

*Fig.5*

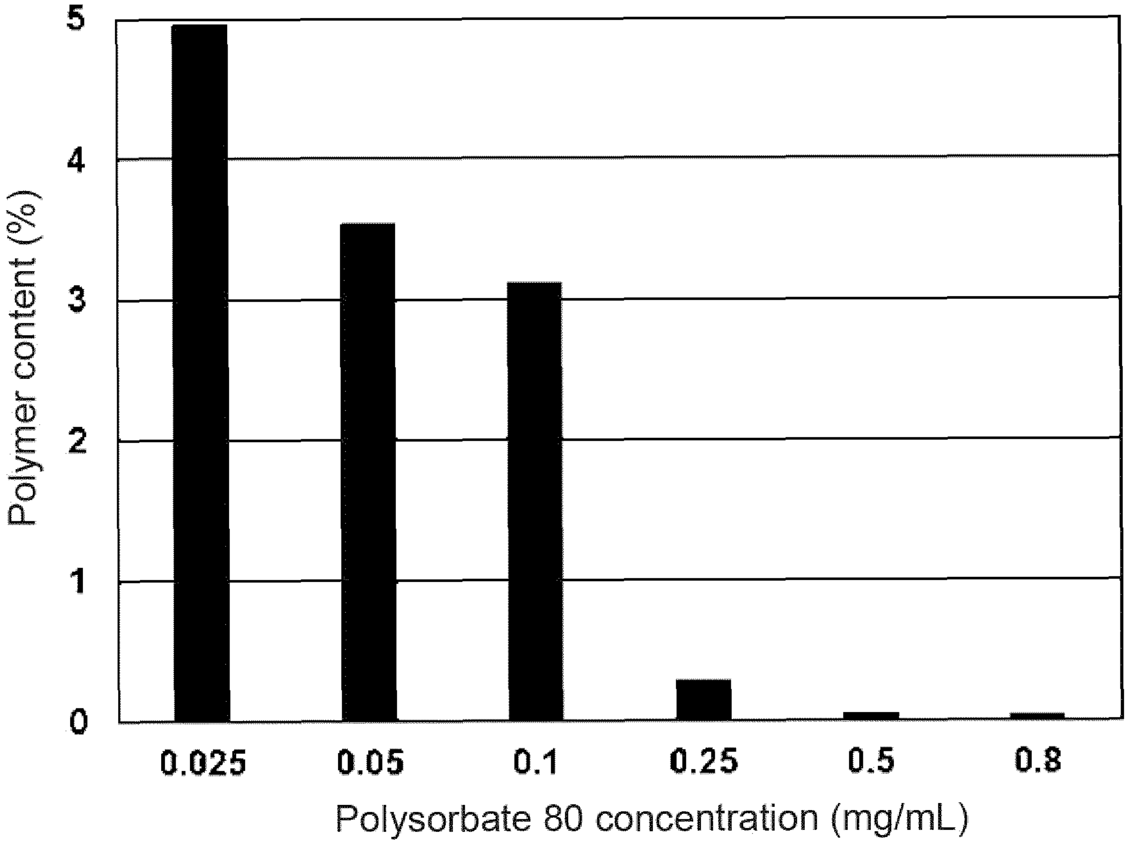
Polymer content (%)
5
4
3
2
1
0
0.025
0.05
0.1
0.25
0.5
0.8
Polysorbate 80 concentration (mg/mL)

*Fig.6*

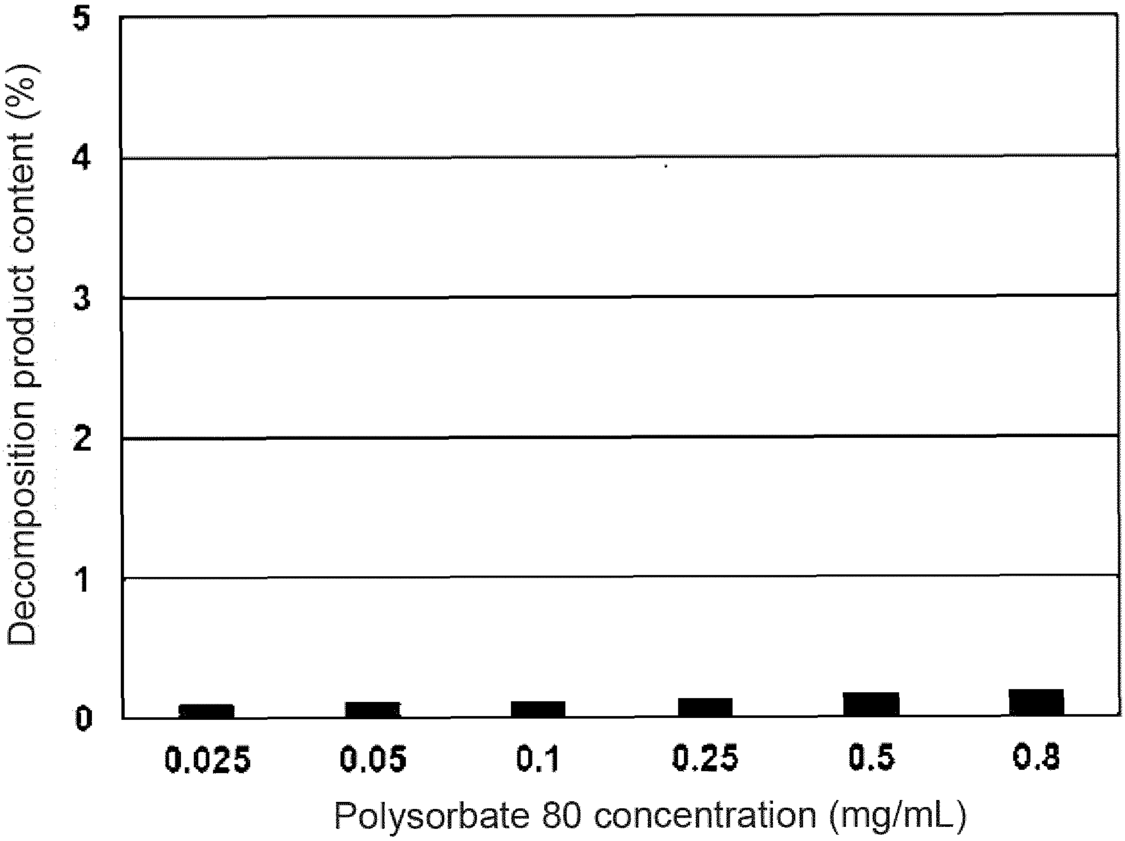
5
4
3
2
1
0
Decomposition product content (%)
0.025
0.05
0.1
0.25
0.5
0.8
Polysorbate 80 concentration (mg/mL)

*Fig.7*

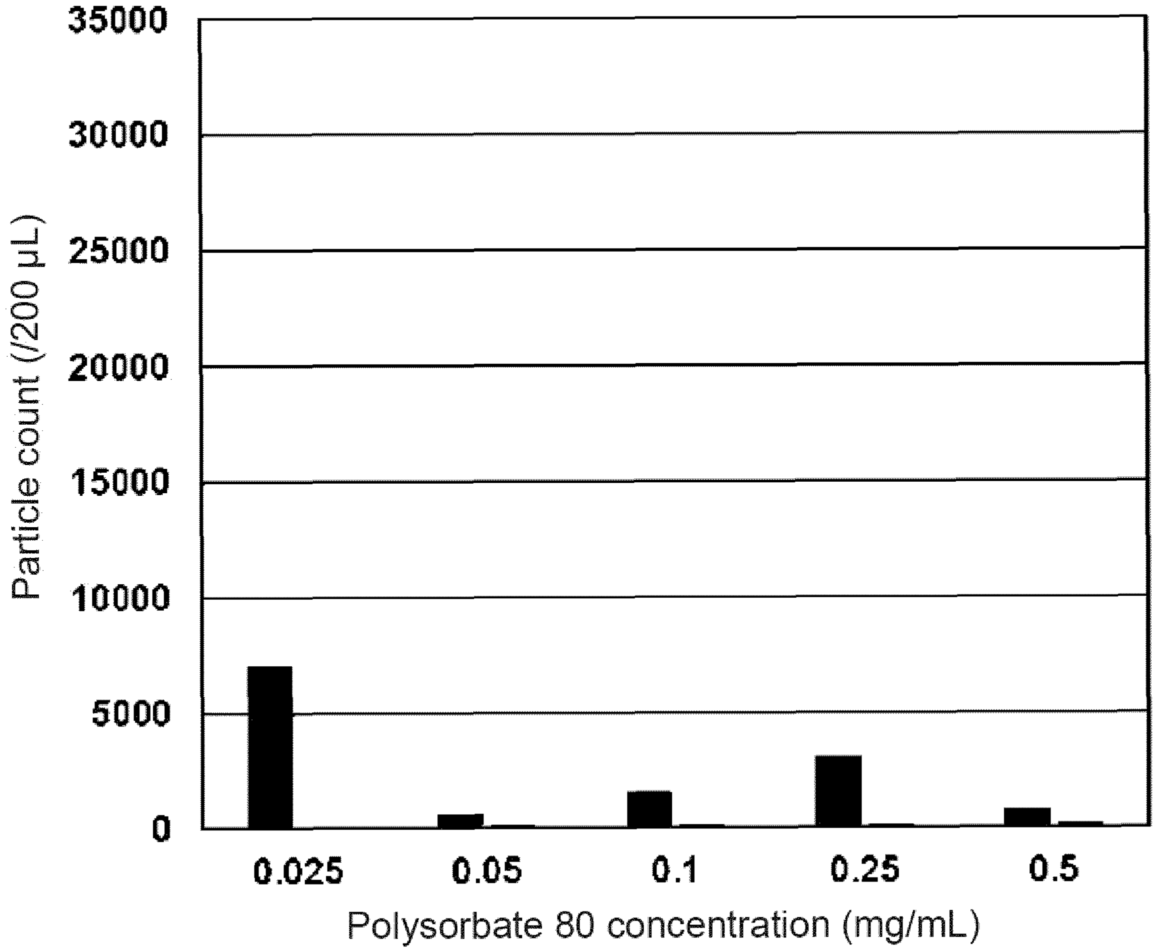
35000
30000
25000
20000
15000
10000
5000
0
Particle count (/200 µL)
0.025
0.05
0.1
0.25
0.5
Polysorbate 80 concentration (mg/mL)

*Fig.8*

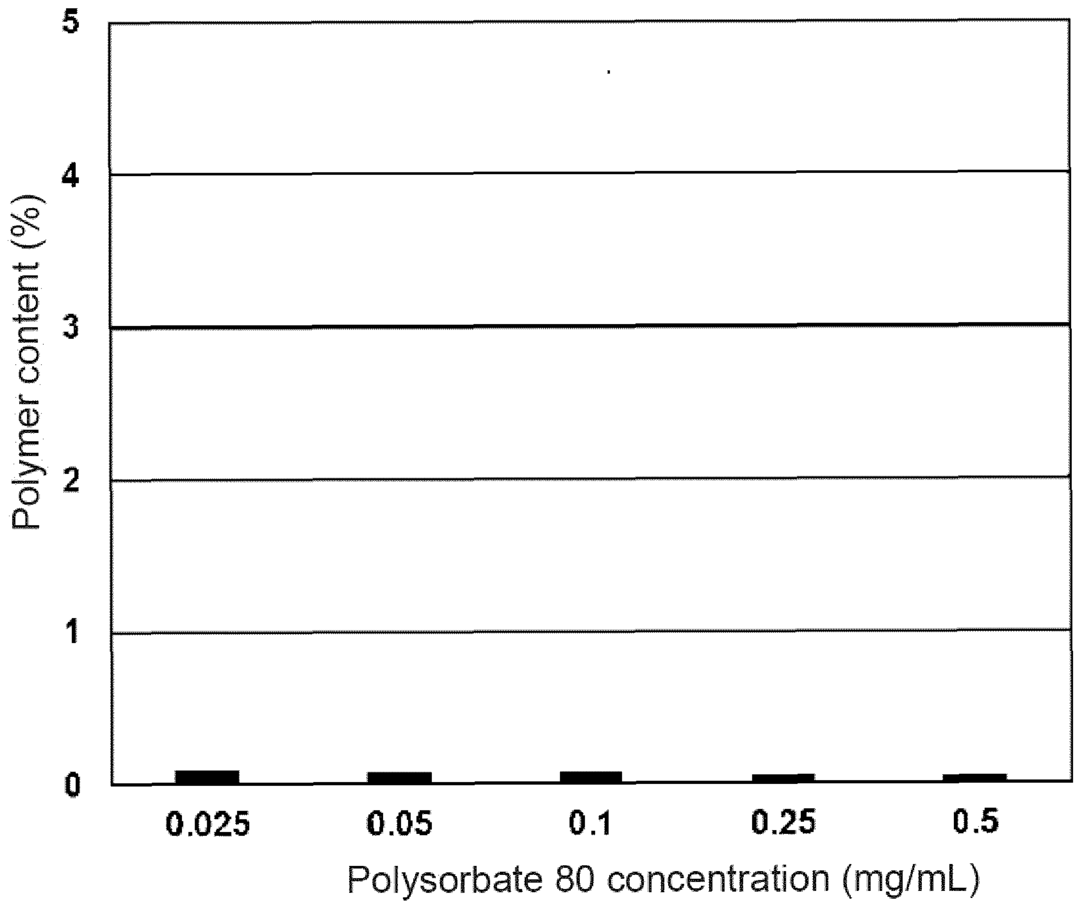
5
4
3
2
1
0
Polymer content (%)
0.025
0.05
0.1
0.25
0.5
Polysorbate 80 concentration (mg/mL)

*Fig.9*

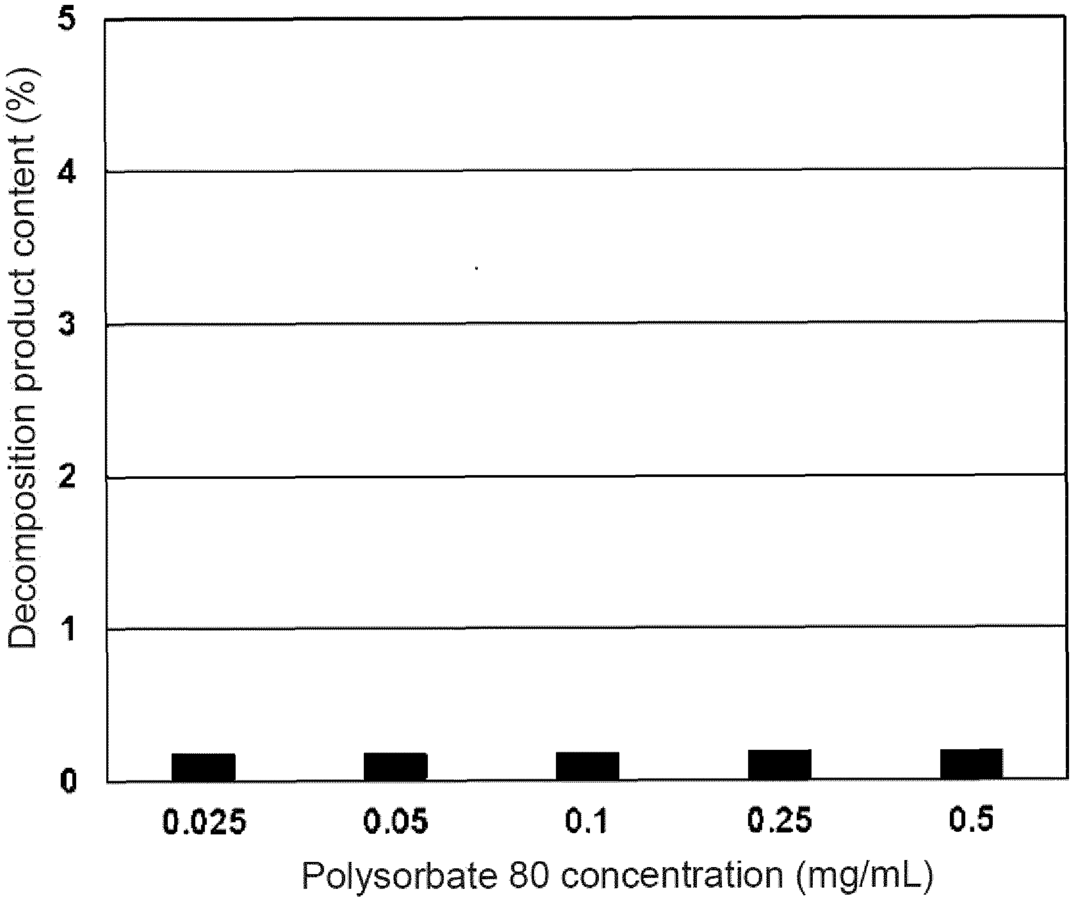
Decomposition product content (%)
5
4
3
2
1
0
0.025
0.05
0.1
0.25
0.5
Polysorbate 80 concentration (mg/mL)

## INTERNATIONAL SEARCH REPORT

International application No. **PCT/JP2022/013755**

**A. CLASSIFICATION OF SUBJECT MATTER**

***A61K 38/43***(2006.01)i; ***A61J 1/05***(2006.01)i; ***A61K 9/08***(2006.01)i; ***A61K 9/19***(2006.01)i; ***A61K 38/47***(2006.01)i; ***A61K 39/395***(2006.01)i; ***A61K 45/00***(2006.01)i; ***A61K 47/02***(2006.01)i; ***A61K 47/10***(2006.01)i; ***A61K 47/12***(2006.01)i; ***A61K 47/18***(2006.01)i; ***A61K 47/26***(2006.01)i; ***A61K 47/34***(2017.01)i; ***A61K 47/65***(2017.01)i; ***A61K 47/68***(2017.01)i; ***A61P 3/00***(2006.01)i; ***A61P 43/00***(2006.01)i; ***C07K 16/28***(2006.01)i; ***C07K 16/46***(2006.01)i; ***C12N 9/24***(2006.01)i; ***C12N 15/13***(2006.01)i; ***C12N 15/56***(2006.01)i; ***C12N 15/62***(2006.01)i

FI: A61K38/43; A61J1/05 311; A61K9/08; A61K9/19; A61K38/47; A61K39/395 C; A61K39/395 D; A61K39/395 L; A61K39/395 N; A61K45/00 ZNA; A61K47/02; A61K47/10; A61K47/12; A61K47/18; A61K47/26; A61K47/34; A61K47/65; A61K47/68; A61P3/00; A61P43/00 111; C07K16/28; C07K16/46; C12N9/24; C12N15/13; C12N15/56; C12N15/62 Z

According to International Patent Classification (IPC) or to both national classification and IPC

**B. FIELDS SEARCHED**

Minimum documentation searched (classification system followed by classification symbols)

A61K38/43; A61J1/05; A61K9/08; A61K9/19; A61K38/47; A61K39/395; A61K45/00; A61K47/02; A61K47/10; A61K47/12; A61K47/18; A61K47/26; A61K47/34; A61K47/65; A61K47/68; A61P3/00; A61P43/00; C07K16/28; C07K16/46; C12N9/24; C12N15/13; C12N15/56; C12N15/62

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 10-513433 A (XOMA CORPORATION) 22 December 1998 (1998-12-22) claims 1, 3, 5, p. 4, line 7 to p. 5, line 3, p. 7, lines 7-23, p. 36, line 5 from the bottom to p. 37, line 4, example 2, experiment 52-58, examples 3-6 | 1-3, 5-9, 11, 15, 16 |
| Y | | 1-43 |
| X | JP 2020-506955 A (JOINT STOCK COMPANY "BIOCAD") 05 March 2020 (2020-03-05) claims 1, 5, 6, 9-11, paragraphs [0052], [0076], [0087], examples 5, #9, #16, #25, #32, #42, #49 | 1-3, 5-7, 10, 11, 15-17, 38 |
| Y | | 1-43 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No. **PCT/JP2022/013755**

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2010-525034 A (NOVO NORDISK HEALTH CARE AG) 22 July 2010 (2010-07-22) claims 1, 7-12, 16, paragraph [0027], example 7 | 1-3, 5, 9, 10, 15, 16, 38<br>1-43 |
| Y | JP 2019-048800 A (JCR PHARMACEUTICALS CO., LTD.) 28 March 2019 (2019-03-28) claims, examples, etc. | 18-43 |
| Y | WO 2018/124277 A1 (JCR PHARMACEUTICALS CO., LTD.) 05 July 2018 (2018-07-05) claims, examples, etc. | 18-43 |

Form PCT/ISA/210 (second sheet) (January 2015)

INTERNATIONAL SEARCH REPORT

International application No.
PCT/JP2022/013755

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2022/013755**

| Patent document cited in search report | Publication date (day/month/year) | Patent family member(s) | Publication date (day/month/year) |
|---|---|---|---|
| JP 10-513433 A | 22 December 1998 | US 5488034 A<br>claims 1-5, column 1, lines 9-34, column 2, line 58 to column 3, line 25, examples<br>WO 1994/017819 A1<br>EP 682524 A1<br>CN 1127992 A | |
| JP 2020-506955 A | 05 March 2020 | WO 2018/124948 A1<br>EP 3563867 A1<br>claims 1, 5, 6, 9-11, paragraphs [0063], [0091], [0102], examples 5, #9, #16, #25, #32, #42, #49<br>RU 2016152691 A<br>KR 10-2019-0104043 A<br>CN 110536698 A | |
| JP 2010-525034 A | 22 July 2010 | US 2010/0294677 A1<br>claims 1, 7-12, 16, paragraph [0097], example 7<br>WO 2008/135500 A1<br>EP 2152233 A1<br>CN 101674805 A | |
| JP 2019-048800 A | 28 March 2019 | US 2021/0061918 A1<br>claims, examples, etc.<br>WO 2019/049967 A1<br>EP 3679945 A1<br>KR 10-2020-0051738 A<br>CN 111278453 A | |
| WO 2018/124277 A1 | 05 July 2018 | US 2019/0336586 A1<br>claims, examples, etc.<br>EP 3563863 A1<br>CN 110114078 A<br>KR 10-2019-0102012 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6279466 B


### Non-patent literature cited in the description


- **ALTSCHUL SF.** *J Mol. Biol.,* 1990, vol. 215, 403-10
- **PEARSON ; LIPMAN.** *Proc. Natl. Acad. Sci. USA.,* 1988, vol. 85, 2444
- **SMITH ; WATERMAN.** *Adv. Appl. Math,* 1981, vol. 2, 482-9
- **MIYAHARA M.** *J. Biol. Chem.,* 2000, vol. 275, 613-618
- **MIYAHARA M.** *J. Biol. Chem.,* 2000, vol. 275, 613-618